# EUROPEAN PATENT APPLICATION

(11) **EP 4 386 084 A1**
(43) Date of publication of application: **19.06.2024**
(21) Application number: 22213328.2
(22) Date of filing: 14.12.2022
(51) Int. Cl.: C12N 9/48, A61K 38/00, A61K 47/68, A61P 11/00, A61P 31/14, C07K 14/705

(54) **IMPROVED ACE2 FUSION PROTEINS**

(71) Applicant: Formycon AG, 82152 Planegg (DE)
(72) Inventor: REITER, Alwin, 80337 München (DE); BROCKMEYER, Carsten, 85417 Marzling (DE); WOLSCHIN, Florian, 81667 München (DE)
(74) Representative: Maiwald GmbH

(57) **Abstract**

The present invention relates to fusion proteins of ACE2 with IgG Fc and the medical use of these fusion proteins, in particular in the prevention or treatment of infections with coronaviruses such as SARS-CoV-2, as well as methods of producing them.

## Description

### FIELD OF THE INVENTION

The present invention relates to fusion proteins of ACE2 with IgG Fc and the medical use of these fusion proteins, in particular in the prevention or treatment of infections with coronaviruses such as SARS-CoV-2, as well as methods of producing them.

### BACKGROUND OF THE INVENTION

ACE2 (angiotensin converting enzyme 2) is a key metalloprotease of the renin-angiotensin system with a catalytic zinc atom in the centre (Donoghue et al. (2002) Circ. Res. 87: e1-e9). Full-length ACE2 consists of an N-terminal extracellular peptidase domain, a collectrin-like domain, a single transmembrane helix and a short intracellular segment. It acts to cleave Angiotensin II to produce Angiotensin (1-7) and Angiotensin I to produce Angiotensin (1-9) which is then processed by other enzymes to become Angiotensin (1-7). ACE2 acts to lower the blood pressure and counters the activity of ACE in order to maintain a balance in the Ras/MAS system. Accordingly, it is a promising target for the treatment of cardiovascular diseases.

Recently, ACE2 has gained much attention as a receptor for coronaviruses and in particular the novel coronavirus SARS-CoV-2. SARS-CoV-2 is a coronavirus which was first discovered in December 2019 in Wuhan, China, but spread rapidly all over the world, leading to a world-wide pandemic. On 19 November 2021, the Johns Hopkins University counted more than 638 millions of confirmed infections worldwide, causing the death of more than 6.5 million people. The pandemic led to a lock-down in many countries with very significant economic and social effects and despite the availability of vaccines is still a constant concern in many countries.

It was shown that ACE2 functions as a receptor for SARS-CoV (Li et al. (2003) Nature 426: 450-454; Prakabaran et al. (2004) Biochem. Biophys. Res. Comm. 314: 235-241) and for SARS-CoV-2 (Yan et al. (2020) Science 367: 1444-1485). Further, entry of SARS-CoV-2 into the respiratory cells depends on ACE2 and the serine protease TMPRSS2 (Hoffmann et al. (2020) Cell 181: 1-10).

In view of the important role of ACE2 for virus entry into the cell, it was proposed to use soluble ACE2 for blocking SARS binding to the cell (WO 2005/032487; WO 2006/122819). The same approach was also suggested for the treatment of infections with SARS-CoV-2 (Kruse (2020) F1000Res. 9:72). Clinical trials with a soluble form of ACE2 in the treatment of infections with SARS-CoV-2 have been initiated by the company Apeiron (Pharmazeutische Zeitung, 10 April 2020) and the first results showed that one patient with severe Covid-19 caused by SARS-CoV-2 recovered fast upon treatment with soluble ACE2 (Zoufaly et al. (2020) The Lancet Respiratory Medicine 8: 1154-1158, available at https://doi.org/10.1016/S2213-2600(20)30418-5).

However, isolated receptor domains are typically characterized by a low stability and plasma half-life. For the soluble form of ACE2 a dose-dependent terminal half-life of 10 hours was shown (Haschke et al. (2013) Clin. Pharmacokinet. 52: 783-792). In view of these results, it was decided to administer the soluble form of ACE2 as twice-daily infusion in a later study (Khan et al. (2017) Critical Care 21: 234). However, an administration of more than one time per day is inconvenient both for the patient and the medical personnel.

A fusion protein of ACE2 consisting of the extracellular domain of either enzymatically active or enzymatically inactive ACE2 linked to the Fc domain of human IgG1 was constructed and tested. It was shown that both constructs potently neutralized both SARS-CoV and SARS-CoV-2 and inhibited S (Spike) protein-mediated fusion (Lei et al. (2020) Nature Communications 11: 2070). Further, a fusion protein called COVIDTRAPTM or STI-4398 was developed for clinical testing by the company Sorrento Therapeutics (see https://www.globenewswire.com/news-release/2020/03/20/2003957/0/en/SORRENTO-DEVELOPS-STI-4398-COVIDTRAP-PROTEIN-FOR-POTENTIAL-PREVENTION-AND-TREATMENT-OF-SARS-COV-2-CORONAVIRUS-DISEASE-COVID-19.html). Liu et al. (2020) Int. J. Biol. Macromol. 165: 1626-1633 describe fusion proteins of wild-type ACE2 and nine ACE2 mutants affecting catalytic activity of ACE2 with the Fc region of human IgG1. However, the interaction of the Fc domain of human IgG1 with Fc gamma receptors on immune cells may enhance the virus infection (Perlman and Dandekar (2005) Nat. Rev. Immunol. 5(12): 917-927; Chen et al. (2020) Current Tropical Medicine Reports 3:1-4).

Tada et al. (2020) Cell Reports 33(12): 18528 disclose an "ACE2 microbody" in which the extracellular domain of catalytically inactive ACE2 is fused to Fc domain 3 of the immunoglobulin heavy chain.

Svilenov et al. (2020), available at Efficient inhibition of SARS-CoV-2 strains by a novel ACE2-lgG4-Fc fusion protein with a stabilized hinge region | bioRxiv, Svilenov et al. (2021) Antiviral Research 196: 105197, WO 2021/234160, WO 2022/090469 and PCT/EP2022/083094 describe ACE2 fusion proteins with the Fc domain of IgG4 and IgG1.

PCT/EP2022/071308 discloses ACE2 fusion proteins with the Fc domain of IgM and IgG2.

WO 2021/170113, WO 2021/183404, US 2021/0284716, WO 2021/217120,

WO 2021/213437 and WO 2021/189772 also disclose fusion proteins of ACE2 with the Fc part of different immunoglobulins.

Nevertheless, there is still a need for agents which can be used for the treatment and/or prevention of infections with coronaviruses, in particular with SARS-CoV-2.

### SUMMARY OF THE INVENTION

The present invention provides a fusion protein comprising:
(a) a first part comprising a variant of a fragment of human ACE2, wherein the fragment of human ACE2 consists of the amino acid sequence according to SEQ ID NO: 1 and wherein the variant of the human ACE2 fragment comprises one or more mutations at an amino acid position selected from the group consisting of T92, Q325 and T27, the numbering referring to SEQ ID NO: 67 and
(b) a second part comprising an Fc portion of a human antibody or a fragment or variant of the Fc portion.

In one embodiment, the one or more mutations are selected from the group consisting of T92I, Q325P and T27A, the numbering referring to SEQ ID NO: 67.

In one embodiment, the variant of the human ACE2 fragment further comprises a mutation at amino acid position S645, the numbering referring to SEQ ID NO: 67.

In one embodiment, the mutation at amino acid position S645 is S645C, the numbering referring to SEQ ID NO: 67.

In one embodiment, the first part of the fusion protein consists of the amino acid sequence according to any one of SEQ ID NOs: 13, 14, 15, 16, 17, 18, 19, 20, 21, 22, 23, 24, 25 or 26.

In one embodiment, the first part of the fusion protein is N-glycosylated and 70% to 95% of the N-glycans on the first part have at least one sialic acid molecule attached thereto.

In one embodiment, the second part of the fusion protein has an amino acid sequence according to any one of SEQ ID NOs: 2, 3, 4, 5, 6 or 7.

In one embodiment, the second part of the fusion protein has an amino acid sequence according to any one of SEQ ID NOs: 2, 6 or 7 and the first and the second part of the fusion protein are linked by by the amino acid sequence according to SEQ ID NO: 12.

In one embodiment, the second part of the fusion protein has an amino acid sequence according to any one of SEQ ID NOs: 3, 4 or 5 and the first and the second part of the fusion protein are linked by by the amino acid sequence according to SEQ ID NO: 11.

In one embodiment, the Fc portion of a human antibody or fragment or variant thereof is afucosylated.

In one embodiment, the Fc part of the fusion protein is N-glycosylated and 15% to 35% of the N-glycans on the Fc part have at least one sialic acid molecule attached thereto.

In one embodiment, the fusion protein has an amino acid sequence according to any one of SEQ ID NOs: 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 68, 69, 70, 71, 72, 73, 74, 75, 76, 77, 78, 79, 80, 81, 82, 83, 84, 85, 86 or 87.

The present invention also relates to a nucleic acid molecule comprising a nucleic acid sequence encoding the fusion protein according to the present invention and to an expression vector comprising said nucleic acid molecule and to a host cell comprising said nucleic acid molecule or said expression vector.

The present invention also relates to a method for producing the fusion protein according to the present invention, comprising culturing said host cell in a suitable culture medium.

In one embodiment said host cell is a CHO cell.

The present invention also relates to the fusion protein of the present invention for medical use.

The present invention also relates to the fusion protein of the present invention for use in preventing and/or treating an infection with a coronavirus binding to ACE2, preferably wherein the coronavirus binding to ACE2 is selected from the group consisting of SARS, SARS-CoV-2 and NL63, preferably it is SARS-CoV-2.

The present invention also relates to a pharmaceutical composition comprising the fusion protein of the present invention and a pharmaceutically acceptable carrier or excipient.

### DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments, but the invention is not limited thereto, but only by the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. a cell or organism is defined to be obtainable by a specific method, this is also to be understood to disclose a cell or organism which is obtained by this method.

Where an indefinite or definite article is used when referring to a singular noun, e.g. "a", "an" or "the", this includes a plural of that noun unless something else is specifically stated.

The present invention relates to a fusion protein comprising a fragment of human ACE2 consisting of amino acids 18 to 732 of the mature form of human ACE2 with one or more mutations at positions 27, 92 and 325 and the Fc part of a human antibody or a variant or fragment thereof.

It was shown that a fragment of human ACE2 comprising amino acids 18 to 732 provides a higher yield and a lower amount of high molecular weight species as well as no non-natural O-glycosylation, i.e. an O-glycosylation which only occurs in recombinantly produced ACE2, but not in ACE2 produced within the human body. Moreover, the affinity of the fragment of human ACE2 comprising amino acids 18 to 732 to the Spike protein of SARS-CoV-2 is higher when compared to the affinity of a fragment of human ACE2 comprising amino acids 18 to 740. It is assumed that this higher affinity is due to a lower flexibility of the ACE2 fragment and consequently a higher tendency to form dimers.

In view of the literature (e.g. Tada et al. (2020) Cell Reports 33(12): 18528) it is assumed that a fusion protein of ACE2 with the Fc portion of a human antibody has a higher affinity to the Spike protein of SARS-CoV-2 than a soluble ACE2 dimer without the Fc portion. It is also assumed that the disulfide bridge between the Fc portions stabilizes the ACE2-dimer formation and shifts the equilibrium to the dimer state, which enhances the binding of the fusion proteins to their target, such as the Spike protein of SARS-CoV-2. The fusion protein of the present invention binds to FcRn which leads to a longer half-life period than the soluble ACE2 dimer.

A "fusion protein" is a protein which is formed by at least two polypeptide parts which are not naturally linked with each other. The two polypeptide parts are linked by a peptide bond and optionally a linker molecule is inserted between the two polypeptide parts. The two polypeptide parts are transcribed and translated as a single molecule. The fusion protein typically has functionalities derived from both polypeptide parts. In the context of the present invention, the fusion protein retains the binding properties of ACE2, in particular the binding of viruses such as coronaviruses, and the increased half-life and Fc receptor binding conferred by the Fc portion of human IgG.

The term "human ACE2" refers to angiotensin converting enzyme 2 derived from a human subject. The full-length sequence of human ACE2 has 805 amino acids. It comprises a signal peptide, an N-terminal extracellular peptidase domain followed by a collectrin-like domain, a single transmembrane helix and a short intracellular segment. The full-length sequence of human ACE2 is depicted in SEQ ID NO: 67. Unless indicated otherwise, the amino acid numbering used herein refers to the numbering of the full-length sequence of human ACE2 according to SEQ ID NO: 67. The extracellular domain of human ACE2 consists of the amino acids 18 to 740 of SEQ ID NO: 67.

The term "fragment of human ACE2" refers to a polypeptide which lacks one or more amino acids compared to the full-length sequence of human ACE2 according to SEQ ID NO:67. The fragment of human ACE2 is capable of binding to the S protein of at least one coronavirus, in particular to the S protein of SARS-CoV-2. The binding of a fragment of human ACE2 to the S protein of at least one coronavirus can be determined in an ELISA assay in which the S protein is immobilized on a substrate and contacted with the fragment of human ACE2 and the interaction between the S protein and the fragment of human ACE2 is detected. Alternatively, the binding of a fragment of human ACE2 to the S protein of at least one coronavirus can be determined by a pseudovirus neutralization assay and the affinity constant KD is proportional to the IC50 value as shown in the following equation: Kᵢ = IC₅₀/(1 + ([L]/K_{D})),. In still another alternative, the binding of a fragment of human ACE2 to the S protein of at least one coronavirus can be determined by surface plasmon resonance, e.g. as described in Shang et al. (2020) Nature doi: 10.1038/s41586-020-2179-y; Wrapp et al. (2020) Science 367(6483): 1260-1263; Lei et al. (2020) Nature Communications 11(1): 2070. In a further alternative, the binding of a fragment of human ACE2 to the S protein of at least one coronavirus can be determined by biolayer interferometry, e.g. as described in Seydoux et al. (2020) https://doi.org/10.1101/2020.05.12.091298. Suitable methods for determining the binding to the S protein are also described in the examples section herein.

In the context of the present invention, the fragment of human ACE2 consists of the amino acid sequence according to SEQ ID NO:1. The amino acid sequence according to SEQ ID NO: 1 starts at amino acid Q18 and ends with amino acid G732 in the sequence according to SEQ ID NO: 67. The amino acid glycine at the C-terminal end of this fragment provides a high rotational freedom which favors the fusion of the two protein parts and increases the stability of the fusion protein. Additionally, it has surprisingly been found that the use of an ACE2 fragment comprising the amino acid sequence which starts at amino acid Q18 and ends with amino acid G732 in the sequence according to SEQ ID NO: 67 provides a better yield as well as a higher stability than a longer ACE2 fragment. Moreover, it lacks a non-natural O glycosylation and neutralizes different SARS-CoV2 variants with higher efficiency than the longer ACE2 fragment, which starts at Q18 and ends at S740 according to SEQ ID NO: 67. In addition, an even shorter ACE2 fragment comprising only amino acids 18 to 615 of ACE2 inhibits infection with SARS-CoV2 even less efficiently than the longer ACE2 fragment comprising amino acids 18 to 740 of ACE2 (Li et al. (2020) J. Virol. 94(22): e01283-20. With regard to neutralization potency, an ACE2 fragment comprising amino acids 18 to 732 inhibits infection with SARS-CoV2 with higher efficiency when compared to an ACE2 fragment comprising amino acids 18 to 740 or 18 to 615.

In one embodiment, the fragment of human ACE2 is N-glycosylated at at least one amino acid residue selected from N53, N90, N103, N322, N432, N546 and N690, the numbering referring to SEQ ID NO: 67. In one embodiment, the fragment of human ACE2 is N-glycosylated at amino acid residues N53, N90 and N322, the numbering referring to SEQ ID NO: 1. In one embodiment, the fragment of human ACE2 is N-glycosylated at amino acid residues N53, N90, N103, N322, N432, N546 and N690, the numbering referring to SEQ ID NO: 67.

In one embodiment, the fragment of human ACE2 consists of the amino acid sequence according to SEQ ID NO:1 and is N-glycosylated at at least one amino acid residue selected from N53, N90, N103, N322, N432, N546 and N690, the numbering referring to SEQ ID NO: 67. In one embodiment, the fragment of human ACE2 consists of the amino acid sequence according to SEQ ID NO:1 and is N-glycosylated at amino acid residues N53, N90 and N322, the numbering referring to SEQ ID NO: 67. In one embodiment, the fragment of human ACE2 consists of the amino acid sequence according to SEQ ID NO:1 and is N-glycosylated at amino acid residues N53, N90, N103, N322, N432, N546 and N690, the numbering referring to SEQ ID NO: 67.

In another embodiment, the fragment of human ACE2 consists of the amino acid sequence according to SEQ ID NO:1 and comprises a mutation to remove at least one N-glycosylation site selected from N53, N90, N103, N322, N432, N546 and N690, the numbering referring to SEQ ID NO: 67. In one embodiment, the fragment of human ACE2 consists of the amino acid sequence according to SEQ ID NO:1 and comprises a mutation to remove the N-glycosylation site at amino acid residue N103. In one embodiment, the fragment of human ACE2 consists of the amino acid sequence according to SEQ ID NO:1 and comprises a mutation to remove the N-glycosylation site at amino acid residue N546. In one embodiment, the fragment of human ACE2 consists of the amino acid sequence according to SEQ ID NO:1 and comprises a mutation to remove the N-glycosylation site at amino acid residue N432. In one embodiment, the fragment of human ACE2 consists of the amino acid sequence according to SEQ ID NO:1 and comprises a mutation to remove the N-glycosylation site at amino acid residues N103 and N546. In one embodiment, the mutation to remove at least one N-glycosylation site is a mutation from asparagine to an amino acid selected from alanine, glutamine, glycine, leucine and isoleucine.

In one embodiment, the fragment of human ACE2 consists of the amino acid sequence according to SEQ ID NO:1 and comprises at least one mutation selected from the group consisting of N53A, N90A, N103A, N322A, N432A, N546A and N690A, the numbering referring to SEQ ID NO: 67. In one embodiment, the fragment of human ACE2 consists of the amino acid sequence according to SEQ ID NO:1 and comprises the mutation N103A, the numbering referring to SEQ ID NO: 67. In one embodiment, the fragment of human ACE2 consists of the amino acid sequence according to SEQ ID NO:1 and comprises the mutation N546A, the numbering referring to SEQ ID NO: 67. In one embodiment, the fragment of human ACE2 consists of the amino acid sequence according to SEQ ID NO:1 and comprises the mutation N432A, the numbering referring to SEQ ID NO: 67. In one embodiment, the fragment of human ACE2 consists of the amino acid sequence according to SEQ ID NO:1 and comprises the mutations N103A and N546A, the numbering referring to SEQ ID NO: 67. In one embodiment, the fragment of human ACE2 consists of the amino acid sequence according to SEQ ID NO:1 and comprises the mutations N53A, N90A, N103A, N322A, N432A, N546A and N690A, the numbering referring to SEQ ID NO: 67.

The aim of mutating one or more N-glycosylation sites is to remove N-glycans with a low level of sialylation and thereby to increase the half-life of the fusion protein.

In the context of the present invention, the term "fragment of human ACE2 consists of/consisting of a protein having the amino acid sequence according to SEQ ID NO: X" is intended to mean that the fragment has exactly the length as defined by the corresponding sequence in the sequence listing and does not comprise any amino acid additions and/or deletions. However, this term does not exclude the presence of amino acid substitution(s) within the sequence of the defined fragment.

In one embodiment, the fragment of human ACE2 comprises or is identified by the amino acid sequence according to SEQ ID NO:1. The amino acid sequence according to SEQ ID NO: 1 starts at amino acid Q18 and ends with amino acid G732 in the sequence according to SEQ ID NO: 67. The amino acid glycine at the C-terminal end of this fragment provides a high rotational freedom which favours the fusion of the two protein parts and increases the stability of the fusion protein. Additionally, the use of an ACE2 fragment comprising or which is identified by the amino acid sequence which starts at amino acid Q18 and ends with amino acid G732 in the sequence according to SEQ ID NO: 67 provides a better yield and a stronger binding to the spike protein of a coronavirus such as SARS-CoV2 than a longer ACE2 fragment.

In one embodiment, the fragment of human ACE2 is N-glycosylated at at least one amino acid residue selected from N53, N90, N103, N322, N432, N546 and N690, the numbering referring to SEQ ID NO: 67. In one embodiment, the fragment of human ACE2 is N-glycosylated at amino acid residues N53, N90 and, N322, the numbering referring to SEQ ID NO: 67. In one embodiment, the fragment of human ACE2 is N-glycosylated at amino acid residues N53, N90, N103, N322, N432, N546 and N690, the numbering referring to SEQ ID NO: 67.

In one embodiment, the fragment of human ACE2 comprises or is identified by the amino acid sequence according to SEQ ID NO:1 and is N-glycosylated at at least one amino acid residue selected from N53, N90, N103, N322, N432, N546 and N690, the numbering referring to SEQ ID NO: 67. In one embodiment, the fragment of human ACE2 comprises or is identified by of the amino acid sequence according to SEQ ID NO:1 and is N-glycosylated at amino acid residues N53, N90 and N322, the numbering referring to SEQ ID NO: 67. In one embodiment, the fragment of human ACE2 comprises or is identified by the amino acid sequence according to SEQ ID NO:1 and is N-glycosylated at amino acid residues N53, N90, N103, N322, N432, N546 and N690, the numbering referring to SEQ ID NO: 67.

The term "N-glycosylated" or "N-glycosylation" means that a glycan structure is attached to the amide nitrogen of an asparagine residue of a protein. A glycan is a branched, flexible chain of carbohydrates and the exact structure of the glycan attached to the asparagine residue of a protein depends on the expression system used for glycoprotein production. Accordingly, the term "N-glycan"means a glycan structure which is attached to the amide nitrogen of an asparagine residue of a protein. In the context of the ACE2 protein, the N - glycans are attached to at least one amino acid residue selected from N53, N90, N103, N322, N432, N546 and N690, the numbering referring to SEQ ID NO: 67, preferably to all amino acid residues N53, N90, N103, N322, N432, N546 and N690, the numbering referring to SEQ ID NO: 67.

According to one aspect of the present invention, 70% to 95%, preferably 73% to 93% and most preferably 85% to 93% of the N-glycans on the ACE2 protein have at least one sialic acid molecule attached to the N-glycan, meaning that one terminal galactose molecule is linked to a sialic acid molecule. The sialic acid molecule is attached to the terminal galactose residues of the glycan structure. In one embodiment, 30% to 60% preferably 33% to 55% and most preferably 35% to 50% of the N-glycans on the ACE2 protein have at least two sialic acid molecules attached to the N-glycan, meaning that two terminal galactose molecules are linked to a sialic acid molecule. In one embodiment, 1% to 12%, preferably 2% to 11%, more preferably 3% to 10% and most preferably 6% to 10% of the N-glycans on the ACE2 protein have at least three sialic acid molecules attached to the N-glycan, meaning that three terminal galactose molecules are linked to a sialic acid molecule. In one embodiment, 0% to 4%, preferably 0.5 % to 3.5%, more preferably 0.8% to 3.3% and most preferably 1% to 3% of the N-glycans on the ACE2 protein have at least four sialic acid molecules attached to the N-glycans, meaning that four terminal galactose molecules are linked to a sialic acid molecule.

In one embodiment, amino acid residues N53, N90, N103, N322, N432, N546 and N690, the numbering referring to SEQ ID NO: 67, are N-glycosylated and 70% to 95%, preferably 73% to 93% and most preferably 85% to 93% of the N-glycans attached to said amino acid residues have at least one sialic acid molecule attached to the N-glycan, meaning that one terminal galactose molecule is linked to a sialic acid molecule. In one embodiment, amino acid residues N53, N90, N103, N322, N432, N546 and N690, the numbering referring to SEQ ID NO: 67, are N-glycosylated and 30% to 60% preferably 33% to 55% and most preferably 35% to 50% of the N-glycans on the ACE2 protein have at least two two sialic acid molecules attached to the N-glycan, meaning that two terminal galactose molecules are linked to a sialic acid molecule. In one embodiment, amino acid residues N53, N90, N103, N322, N432, N546 and N690, the numbering referring to SEQ ID NO: 67, are N-glycosylated and 1% to 12%, preferably 2% to 11%, more preferably 3% to 10% and most preferably 6% to 10% of the N-glycans on the ACE2 protein have at least three sialic acid molecules attached to the N-glycan, meaning that three terminal galactose molecules are linked to a sialic acid molecule. In one embodiment, amino acid residues N53, N90, N103, N322, N432, N546 and N690, the numbering referring to SEQ ID NO: 67, are N-glycosylated and 0% to 4%, preferably 0.5 % to 3.5%, more preferably 0.8% to 3.3% and most preferably 1% to 3% of the N-glycans on the ACE2 protein have at least four sialic acid molecules attached to the N-glycans, meaning that four terminal galactose molecules are linked to a sialic acid molecule.

The glycoprotein structure can be analyzed by different methods known to the skilled person, including digestion of the fusion protein and analysis by peptide mapping. Alternatively, hydrophilic interaction liquid chromatography (HILIC) may be performed for glycoprotein analysis as described in the examples herein.

An increased sialylation leads to an increased half-life of a therapeutic protein (see e.g., Byrne et al. (2007) Drug Discovery Today 12 (7-8): 319-326; Jones et al. (2007) Glycobiology 17(5): 529-540).

The sialylation may be increased by culturing the host cells expressing the fusion protein under specific conditions which increase sialylation as described further below.

As used herein, the term "sialylation" refers to the addition of a sialic acid residue to a protein, including a glycoprotein. Sialic acid is a common name for a family of unique nine-carbon monosaccharides, which can be linked to other oligosaccharides. Two family members are N- acetyl neuraminic acid, abbreviated as Neu5Ac or NANA, and N-glycolyl neuraminic acid, abbreviated as Neu5Gc or NGNA. The most common form of sialic acid in humans is NANA.

A "variant" of the fragment of human ACE2 refers to a fragment as defined above, wherein compared to the corresponding sequence in the amino acid sequence of wild-type, full-length human ACE2 according to SEQ ID NO: 67 at least one amino acid residue is different or at least two, at least three, at least four, at least five, at least six, at least seven, at least eight, at least nine, at least ten, at least eleven or at least thirteen amino acids are different. The "variant" of the fragment of human ACE2 comprises one or more amino acid substitutions in the sequence of the fragment of human ACE2. A "variant" of the fragment of human ACE2 does not comprise any amino acid additions or deletions compared to the sequence from which the variant is derived. In the context of the present invention, the variant of the fragment of human ACE2 is a variant of the fragment of human ACE2 according to SEQ ID NO: 1 and does not comprise any amino acid additions or deletions compared to the sequence according to SEQ ID NO: 1, i.e. it has the same length as the sequence according to SEQ ID NO: 1. Within the scope of the present invention a variant of the fragment of human ACE2 is capable of binding to the S protein of at least one coronavirus, in particular to the S protein of SARS-CoV-2. The binding of a variant of the fragment of human ACE2 to the S protein of at least one coronavirus, in particular to the S protein of SARS-CoV-2, can be determined as described above for fragments of human ACE2.

In one embodiment, the variant of the fragment of human ACE2 differs from the corresponding amino acid sequence in the sequence according to SEQ ID NO: 67 by one amino acid. In another embodiment, the variant of the fragment of human ACE2 differs from the corresponding amino acid sequence in the sequence according to SEQ ID NO: 67 by two amino acids. In still another embodiment, the variant of the fragment of human ACE2 differs from the corresponding amino acid sequence in the sequence according to SEQ ID NO: 67 by three amino acids. In still another embodiment, the variant of the fragment of human ACE2 differs from the corresponding amino acid sequence in the sequence according to SEQ ID NO: 67 by four amino acids. In still another embodiment, the variant of the fragment of human ACE2 differs from the corresponding amino acid sequence in the sequence according to SEQ ID NO: 67 by five amino acids. In still another embodiment, the variant of the fragment of human ACE2 differs from the corresponding amino acid sequence in the sequence according to SEQ ID NO: 67 by six amino acids. In still another embodiment, the variant of the fragment of human ACE2 differs from the corresponding amino acid sequence in the sequence according to SEQ ID NO: 67 by seven amino acids. In still another embodiment, the variant of the fragment of human ACE2 differs from the corresponding amino acid sequence in the sequence according to SEQ ID NO: 67 by eight amino acids. In still another embodiment, the variant of the fragment of human ACE2 differs from the corresponding amino acid sequence in the sequence according to SEQ ID NO: 67 by nine amino acids. In still another embodiment, the variant of the fragment of human ACE2 differs from the corresponding amino acid sequence in the sequence according to SEQ ID NO: 67 by ten amino acids. In still another embodiment, the variant of the fragment of human ACE2 differs from the corresponding amino acid sequence in the sequence according to SEQ ID NO: 67 by eleven amino acids. In still another embodiment, the variant of the fragment of human ACE2 differs from the corresponding amino acid sequence in the sequence according to SEQ ID NO: 67 by twelve amino acids. In still another embodiment, the variant of the fragment of human ACE2 differs from the corresponding amino acid sequence in the sequence according to SEQ ID NO: 67 by thirteen amino acids.

According to the present invention, the variant of the fragment of human ACE2 comprises at least one mutation at an amino acid position selected from T27, T92 and Q325, the numbering referring to SEQ ID NO: 67. It was found that these amino acid residues participate in the interaction with the receptor-binding domain of SARS-CoV2 and that a mutation at one or more of these positions may enhance binding of ACE2 to SARS-CoV2 (Bodie et al. (2022), available at https://doi.org/10.1101/2022.06.23.497326; Suryamohan et al. (2021) Communications Biology 4: 475; Chan et al. (2020) Science 369: 1261-1265).

Preferably, the variant of the fragment of human ACE2 comprises at least one mutation selected from T27A, T92I and Q325P, the numbering referring to SEQ ID NO: 67. In one embodiment, the fragment of human ACE2 consists of the amino acid sequence according to SEQ ID NO: 1 and comprises an amino acid substitution at amino acid residue T27, the numbering referring to SEQ ID NO: 67. In one embodiment, the fragment of human ACE2 consists of the amino acid sequence according to SEQ ID NO: 1 and comprises an amino acid substitution at amino acid residue T92, the numbering referring to SEQ ID NO: 67. In one embodiment, the fragment of human ACE2 consists of the amino acid sequence according to SEQ ID NO: 1 and comprises an amino acid substitution at amino acid residue Q325, the numbering referring to SEQ ID NO: 67.

In one embodiment, the variant of the fragment of human ACE2 consists of the amino acid sequence according to SEQ ID NO: 1 and comprises the amino acid substitution T27A, the numbering referring to SEQ ID NO: 67. The sequence of this variant of the fragment of human ACE2 is shown in SEQ ID NO: 13.

In one embodiment, the variant of the fragment of human ACE2 consists of the amino acid sequence according to SEQ ID NO: 1 and comprises the amino acid substitution T92I, the numbering referring to SEQ ID NO: 67. The sequence of this variant of the fragment of human ACE2 is shown in SEQ ID NO: 14.

In one embodiment, the variant of the fragment of human ACE2 consists of the amino acid sequence according to SEQ ID NO: 1 and comprises the amino acid substitution Q325P, the numbering referring to SEQ ID NO: 67. The sequence of this variant of the fragment of human ACE2 is shown in SEQ ID NO: 15.

In one embodiment, the variant of the fragment of human ACE2 consists of the amino acid sequence according to SEQ ID NO: 1 and comprises the amino acid substitutions T27A and T92I, the numbering referring to SEQ ID NO: 67. The sequence of this variant of the fragment of human ACE2 is shown in SEQ ID NO: 16.

In one embodiment, the variant of the fragment of human ACE2 consists of the amino acid sequence according to SEQ ID NO: 1 and comprises the amino acid substitutions T27A and Q325P, the numbering referring to SEQ ID NO: 67. The sequence of this variant of the fragment of human ACE2 is shown in SEQ ID NO: 17.

In one embodiment, the variant of the fragment of human ACE2 consists of the amino acid sequence according to SEQ ID NO: 1 and comprises the amino acid substitutions T92I and Q325P, the numbering referring to SEQ ID NO: 67. The sequence of this variant of the fragment of human ACE2 is shown in SEQ ID NO: 18.

In one embodiment, the variant of the fragment of human ACE2 consists of the amino acid sequence according to SEQ ID NO: 1 and comprises the amino acid substitutions T27A, T92I and Q325P, the numbering referring to SEQ ID NO: 67. The sequence of this variant of the fragment of human ACE2 is shown in SEQ ID NO: 19.

One variant of the fragment of human ACE2 may be an enzymatically inactive variant. "The enzymatically inactive variant of the fragment of human ACE2" lacks the ability to cleave angiotensin II to Ang1-7. The enzymatic activity of human ACE2 can be determined by methods known to the skilled person. Suitable kits for determining the enzymatic activity of human ACE2 are commercially available, for example from the companies BioVision, Abcam or Anaspec. By using an enzymatically inactive ACE2 variant any side effects associated with the enzymatic activity of ACE2 such as effects on the cardiovascular system or the regulation of blood pressure are eliminated. Further, the risk of counterbalancing the RAS - MAS equilibrium is reduced.

The enzymatically inactive variant of the fragment of human ACE2 may comprise one or more mutations of amino acids within the catalytic center of ACE2. In particular, the enzymatically inactive variant of the fragment of human ACE2 comprises a mutation of the wildtype histidine at residue 374 of the sequence according to SEQ ID NO: 67 and/or a mutation of the wildtype histidine at residue 378 of the sequence according to SEQ ID NO: 67. The wild-type histidine may be mutated to any amino acid other than histidine and particularly, the wild-type histidine is mutated to asparagine. Preferably, the enzymatically inactive variant of the fragment of human ACE2 comprises a H374N and a H378N mutation, the numbering referring to the sequence according to SEQ ID NO: 67.

In another embodiment, the enzymatically inactive variant of the fragment of human ACE2 comprises a mutation at one or more of the following amino acid residues, the numbering referring to the sequence according to SEQ ID NO: 67: residue 345 (histidine in wild-type), 273 (arginine in wild-type), 402 (glutamic acid in wild-type) and 505 (histidine in wild-type). In one embodiment, the enzymatically inactive variant of the fragment of human ACE2 comprises a mutation of histidine at residue 345 to alanine or leucine, a mutation of arginine at residue 273 to alanine, glutamine or lysine, a mutation of glutamic acid at residue 402 to alanine and/or a mutation of histidine at residue 505 to alanine or leucine, the numbering referring to the sequence according to SEQ ID NO: 67. In a particular embodiment, the enzymatically inactive variant of the fragment of human ACE2 comprises a mutation of arginine at residue 273 to alanine (also called R273A mutation). It was found that arginine 273 is critical for substrate binding and that its substitution abolishes enzymatic activity (Guy et al. (2005) FEBS J. 272(14): 3512-3520).

In one embodiment, the fragment of human ACE2 consists of the sequence according to SEQ ID NO: 1 and comprises a T27A mutation, a H374N mutation and a H378N mutation, the numbering referring to SEQ ID NO: 67.

In one embodiment, the fragment of human ACE2 consists of the sequence according to SEQ ID NO: 1 and comprises a T92I mutation, a H374N mutation and a H378N mutation, the numbering referring to SEQ ID NO: 67.

In one embodiment, the fragment of human ACE2 consists of the sequence according to SEQ ID NO: 1 and comprises a Q325P mutation, a H374N mutation and a H378N mutation, the numbering referring to SEQ ID NO: 67.

In one embodiment, the fragment of human ACE2 consists of the sequence according to SEQ ID NO: 1 and comprises a T27A mutation, a T92I mutation, a H374N mutation and a H378N mutation, the numbering referring to SEQ ID NO: 67.

In one embodiment, the fragment of human ACE2 consists of the sequence according to SEQ ID NO: 1 and comprises a T27A mutation, a Q325P mutation, a H374N mutation and a H378N mutation, the numbering referring to SEQ ID NO: 67.

In one embodiment, the fragment of human ACE2 consists of the sequence according to SEQ ID NO: 1 and comprises a T92I mutation, a Q325P mutation, a H374N mutation and a H378N mutation, the numbering referring to SEQ ID NO: 67.

In one embodiment, the fragment of human ACE2 consists of the sequence according to SEQ ID NO: 1 and comprises a T27A mutation, a T92I mutation, a Q325P mutation, a H374N mutation and a H378N mutation, the numbering referring to SEQ ID NO: 67.

In one embodiment, the fragment of human ACE2 comprises or is identified by the sequence according to SEQ ID NO: 1 and comprises a T27A mutation, a H374N mutation and a H378N mutation, the numbering referring to SEQ ID NO: 67.

In one embodiment, the fragment of human ACE2 comprises or is identified by the sequence according to SEQ ID NO: 1 and comprises a T92I mutation, a H374N mutation and a H378N mutation, the numbering referring to SEQ ID NO: 67.

In one embodiment, the fragment of human ACE2 comprises or is identified by the sequence according to SEQ ID NO: 1 and comprises a Q325P mutation, a H374N mutation and a H378N mutation, the numbering referring to SEQ ID NO: 67.

In one embodiment, the fragment of human ACE2 comprises or is identified by the sequence according to SEQ ID NO: 1 and comprises a T27A mutation, a T92I mutation, a H374N mutation and a H378N mutation, the numbering referring to SEQ ID NO: 67.

In one embodiment, the fragment of human ACE2 comprises or is identified by the sequence according to SEQ ID NO: 1 and comprises a T27A mutation, a Q325P mutation, a H374N mutation and a H378N mutation, the numbering referring to SEQ ID NO: 67.

In one embodiment, the fragment of human ACE2 comprises or is identified by the sequence according to SEQ ID NO: 1 and comprises a T92I mutation, a Q325P mutation, a H374N mutation and a H378N mutation, the numbering referring to SEQ ID NO: 67.

In one embodiment, the fragment of human ACE2 comprises or is identified by the sequence according to SEQ ID NO: 1 and comprises a T27A mutation, a T92I mutation, a Q325P mutation, a H374N mutation and a H378N mutation, the numbering referring to SEQ ID NO: 67.

In one embodiment, the fragment of human ACE2 consists of a protein having the amino acid sequence according to SEQ ID NO: 1 and comprises a T27A mutation and a R273A mutation, the numbering referring to SEQ ID NO: 67.

In one embodiment, the fragment of human ACE2 consists of a protein having the amino acid sequence according to SEQ ID NO: 1 and comprises a T92I mutation and a R273A mutation, the numbering referring to SEQ ID NO: 67.

In one embodiment, the fragment of human ACE2 consists of a protein having the amino acid sequence according to SEQ ID NO: 1 and comprises a Q325P mutation and a R273A mutation, the numbering referring to SEQ ID NO: 67.

In one embodiment, the fragment of human ACE2 consists of a protein having the amino acid sequence according to SEQ ID NO: 1 and comprises a T27A mutation, a T92I mutation and a R273A mutation, the numbering referring to SEQ ID NO: 67.

In one embodiment, the fragment of human ACE2 consists of a protein having the amino acid sequence according to SEQ ID NO: 1 and comprises a T27A mutation, a Q325P mutation and a R273A mutation, the numbering referring to SEQ ID NO: 67.

In one embodiment, the fragment of human ACE2 consists of a protein having the amino acid sequence according to SEQ ID NO: 1 and comprises a T92I mutation, a Q325P mutation and a R273A mutation, the numbering referring to SEQ ID NO: 67.

In one embodiment, the fragment of human ACE2 consists of a protein having the amino acid sequence according to SEQ ID NO: 1 and comprises a T27A mutation, a T92I mutation, a Q325P mutation and a R273A mutation, the numbering referring to SEQ ID NO: 67.

In one embodiment, the fragment of human ACE2 comprises or is identified by the sequence according to SEQ ID NO: 1 and comprises a T27A mutation and a R273A mutation, the numbering referring to SEQ ID NO: 67.

In one embodiment, the fragment of human ACE2 comprises or is identified by the sequence according to SEQ ID NO: 1 and comprises a T92I mutation and a R273A mutation, the numbering referring to SEQ ID NO: 67.

In one embodiment, the fragment of human ACE2 comprises or is identified by the sequence according to SEQ ID NO: 1 and comprises a Q325P mutation and a R273A mutation, the numbering referring to SEQ ID NO: 67.

In one embodiment, the fragment of human ACE2 comprises or is identified by the sequence according to SEQ ID NO: 1 and comprises a T27A mutation, a T92I mutation and a R273A mutation, the numbering referring to SEQ ID NO: 67.

In one embodiment, the fragment of human ACE2 comprises or is identified by the sequence according to SEQ ID NO: 1 and comprises a T27A mutation, a Q325P mutation and a R273A mutation, the numbering referring to SEQ ID NO: 67.

In one embodiment, the fragment of human ACE2 comprises or is identified by the sequence according to SEQ ID NO: 1 and comprises a T92I mutation, a Q325P mutation and a R273A mutation, the numbering referring to SEQ ID NO: 67.

In one embodiment, the fragment of human ACE2 comprises or is identified by the sequence according to SEQ ID NO: 1 and comprises a T27A mutation, a T92I mutation, a Q325P mutation and a R273A mutation, the numbering referring to SEQ ID NO: 67.

Another variant of the fragment of human ACE2 may be a variant which provides an additional cysteine for the formation of disulfide bridges between two ACE2 molecules. The disulfide bridge increases the intrinsic stability of the fusion protein and may also have an effect on the binding of the fusion protein to its target. The additional cysteine may be provided by a substitution of serine 645 in the numbering of SEQ ID NO: 67 with cysteine.

In one embodiment, the fragment of human ACE2 consists of a protein having the amino acid sequence according to SEQ ID NO: 1 and comprises a T27A mutation and a S645C mutation, the numbering referring to SEQ ID NO: 67. The sequence of this fragment of human ACE2 is shown in SEQ ID NO: 20.

In one embodiment, the fragment of human ACE2 consists of a protein having the amino acid sequence according to SEQ ID NO: 1 and comprises a T92I mutation and a S645C mutation, the numbering referring to SEQ ID NO: 67. The sequence of this fragment of human ACE2 is shown in SEQ ID NO: 21.

In one embodiment, the fragment of human ACE2 consists of a protein having the amino acid sequence according to SEQ ID NO: 1 and comprises a Q325P mutation and a S645C mutation, the numbering referring to SEQ ID NO: 67. The sequence of this fragment of human ACE2 is shown in SEQ ID NO: 22.

In one embodiment, the fragment of human ACE2 consists of a protein having the amino acid sequence according to SEQ ID NO: 1 and comprises a T27A mutation, a T92I mutation and a S645C mutation, the numbering referring to SEQ ID NO: 67. The sequence of this fragment of human ACE2 is shown in SEQ ID NO: 23.

In one embodiment, the fragment of human ACE2 consists of a protein having the amino acid sequence according to SEQ ID NO: 1 and comprises a T27A mutation, a Q325P mutation and a S645C mutation, the numbering referring to SEQ ID NO: 67. The sequence of this fragment of human ACE2 is shown in SEQ ID NO: 24.

In one embodiment, the fragment of human ACE2 consists of a protein having the amino acid sequence according to SEQ ID NO: 1 and comprises a T92I mutation, a Q325P mutation and a S645C mutation, the numbering referring to SEQ ID NO: 67. The sequence of this fragment of human ACE2 is shown in SEQ ID NO: 25.

In one embodiment, the fragment of human ACE2 consists of a protein having the amino acid sequence according to SEQ ID NO: 1 and comprises a T27A mutation, a T92I mutation, a Q325P mutation and a S645C mutation, the numbering referring to SEQ ID NO: 67. The sequence of this fragment of human ACE2 is shown in SEQ ID NO: 26.

In one embodiment, the fragment of human ACE2 comprises or is identified by the amino acid sequence according to SEQ ID NO: 1 and comprises a T27A mutation and a S645C mutation, the numbering referring to SEQ ID NO: 67. The sequence of this fragment of human ACE2 is shown in SEQ ID NO: 20.

In one embodiment, the fragment of human ACE2 comprises or is identified by the amino acid sequence according to SEQ ID NO: 1 and comprises a T92I mutation and a S645C mutation, the numbering referring to SEQ ID NO: 67. The sequence of this fragment of human ACE2 is shown in SEQ ID NO: 21.

In one embodiment, the fragment of human ACE2 comprises or is identified by the amino acid sequence according to SEQ ID NO: 1 and comprises a Q325P mutation and a S645C mutation, the numbering referring to SEQ ID NO: 67. The sequence of this fragment of human ACE2 is shown in SEQ ID NO: 22.

In one embodiment, the fragment of human ACE2 comprises or is identified by the amino acid sequence according to SEQ ID NO: 1 and comprises a T27A mutation, a T92I mutation and a S645C mutation, the numbering referring to SEQ ID NO: 67. The sequence of this fragment of human ACE2 is shown in SEQ ID NO: 23.

In one embodiment, the fragment of human ACE2 comprises or is identified by the amino acid sequence according to SEQ ID NO: 1 and comprises a T27A mutation, a Q325P mutation and a S645C mutation, the numbering referring to SEQ ID NO: 67. The sequence of this fragment of human ACE2 is shown in SEQ ID NO: 24.

In one embodiment, the fragment of human ACE2 comprises or is identified by the amino acid sequence according to SEQ ID NO: 1 and comprises a T92I mutation, a Q325P mutation and a S645C mutation, the numbering referring to SEQ ID NO: 67. The sequence of this fragment of human ACE2 is shown in SEQ ID NO: 25.

In one embodiment, the fragment of human ACE2 comprises or is identified by the amino acid sequence according to SEQ ID NO: 1 and comprises a T27A mutation, a T92I mutation, a Q325P mutation and a S645C mutation, the numbering referring to SEQ ID NO: 67. The sequence of this fragment of human ACE2 is shown in SEQ ID NO: 26.

In one embodiment, the fragment of human ACE2 consists of a protein having the amino acid sequence according to SEQ ID NO: 1 and comprises a T27A mutation, a H374N mutation, a H378N mutation and a S645C mutation, the numbering referring to SEQ ID NO: 67.

In one embodiment, the fragment of human ACE2 consists of a protein having the amino acid sequence according to SEQ ID NO: 1 and comprises a T92I mutation, a H374N mutation, a H378N mutation and a S645C mutation, the numbering referring to SEQ ID NO: 67.

In one embodiment, the fragment of human ACE2 consists of a protein having the amino acid sequence according to SEQ ID NO: 1 and comprises a Q325P mutation, a H374N mutation, a H378N mutation and a S645C mutation, the numbering referring to SEQ ID NO: 67.

In one embodiment, the fragment of human ACE2 consists of a protein having the amino acid sequence according to SEQ ID NO: 1 and comprises a T27A mutation, a T92I mutation, a H374N mutation, a H378N mutation and a S645C mutation, the numbering referring to SEQ ID NO: 67.

In one embodiment, the fragment of human ACE2 consists of a protein having the amino acid sequence according to SEQ ID NO: 1 and comprises a T27A mutation, a Q325P mutation, a H374N mutation, a H378N mutation and a S645C mutation, the numbering referring to SEQ ID NO: 67.

In one embodiment, the fragment of human ACE2 consists of a protein having the amino acid sequence according to SEQ ID NO: 1 and comprises a T92I mutation, a Q325P mutation, a H374N mutation, a H378N mutation and a S645C mutation, the numbering referring to SEQ ID NO: 67.

In one embodiment, the fragment of human ACE2 consists of a protein having the amino acid sequence according to SEQ ID NO: 1 and comprises a T27A mutation, a T92I mutation, a Q325P mutation, a H374N mutation, a H378N mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a H374N mutation, a H378N mutation and a S645C mutation, the numbering referring to SEQ ID NO: 67.

In one embodiment, the fragment of human ACE2 comprises or is identified by the amino acid sequence according to SEQ ID NO: 1 and comprises a T92I mutation, a H374N mutation, a H378N mutation and a S645C mutation, the numbering referring to SEQ ID NO: 67.

In one embodiment, the fragment of human ACE2 comprises or is identified by the amino acid sequence according to SEQ ID NO: 1 and comprises a Q325P mutation, a H374N mutation, a H378N mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a T92I mutation, a H374N mutation, a H378N mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a Q325P mutation, a H374N mutation, a H378N mutation and a S645C mutation, the numbering referring to SEQ ID NO: 67.

In one embodiment, the fragment of human ACE2 comprises or is identified by the amino acid sequence according to SEQ ID NO: 1 and comprises a T92I mutation, a Q325P mutation, a H374N mutation, a H378N mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a T92I mutation, a Q325P mutation, a H374N mutation, a H378N mutation and a S645C mutation, the numbering referring to SEQ ID NO: 67.

In one embodiment, the fragment of human ACE2 consists of a protein having the amino acid sequence according to SEQ ID NO: 1 and comprises a T27A mutation, a R273A mutation and a S645C mutation, the numbering referring to SEQ ID NO: 67.

In one embodiment, the fragment of human ACE2 consists of a protein having the amino acid sequence according to SEQ ID NO: 1 and comprises a T92I mutation, a R273A mutation and a S645C mutation, the numbering referring to SEQ ID NO: 67.

In one embodiment, the fragment of human ACE2 consists of a protein having the amino acid sequence according to SEQ ID NO: 1 and comprises a R273A mutation, a Q325P mutation and a S645C mutation, the numbering referring to SEQ ID NO: 67.

In one embodiment, the fragment of human ACE2 consists of a protein having the amino acid sequence according to SEQ ID NO: 1 and comprises a T27A mutation, a T92I mutation, a R273A mutation and a S645C mutation, the numbering referring to SEQ ID NO: 67.

In one embodiment, the fragment of human ACE2 consists of a protein having the amino acid sequence according to SEQ ID NO: 1 and comprises a T27A mutation, a R273A mutation, a Q325P mutation, and a S645C mutation, the numbering referring to SEQ ID NO: 67.

In one embodiment, the fragment of human ACE2 consists of a protein having the amino acid sequence according to SEQ ID NO: 1 and comprises a T92I mutation, a R273A mutation, a Q325P mutation and a S645C mutation, the numbering referring to SEQ ID NO: 67.

In one embodiment, the fragment of human ACE2 consists of a protein having the amino acid sequence according to SEQ ID NO: 1 and comprises a T27A mutation, a T92I mutation, a R273A mutation, a Q325P mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a R273A mutation and a S645C mutation, the numbering referring to SEQ ID NO: 67.

In one embodiment, the fragment of human ACE2 comprises or is identified by the amino acid sequence according to SEQ ID NO: 1 and comprises a T92I mutation, a R273A mutation and a S645C mutation, the numbering referring to SEQ ID NO: 67.

In one embodiment, the fragment of human ACE2 comprises or is identified by the amino acid sequence according to SEQ ID NO: 1 and comprises a R273A mutation, a Q325P mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a T92I mutation, a R273A mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a R273A mutation, a Q325P mutation and a S645C mutation, the numbering referring to SEQ ID NO: 67.

In one embodiment, the fragment of human ACE2 comprises or is identified by the amino acid sequence according to SEQ ID NO: 1 and comprises a T92I mutation, a R273A mutation, a Q325P mutation and a S645C mutation, the numbering referring to SEQ ID NO: 67.

In one embodiment, the fragment of human ACE2 comprises or is identified by the amino acid sequence according to SEQ ID NO: 1 and comprises a T27A mutation, a T92I mutation, a R273A mutation, a Q325P mutation and a S645C mutation, the numbering referring to SEQ ID NO: 67.

In one embodiment, the second part of the fusion protein of the present invention comprises the Fc portion of human IgG. The Fc portion of human IgG may be the Fc portion of IgG1, IgG3 or IgG4.

In one embodiment, the second part of the fusion protein of the present invention comprises the Fc portion of human IgG4. The Fc portion of human IgG4 comprises the CH2 and CH3 domains of human IgG4 linked together to form the Fc portion. In a full-length human IgG4 antibody the Fc portion is connected to the Fab fragment through a hinge region. The Fab fragment comprises the heavy chain variable region and the CH1 domain. In one embodiment, the Fc portion of human IgG4 used in the fusion protein of the present invention has the sequence according to SEQ ID NO: 3.

Since the IgG4 subclass of antibodies has only a partial affinity for Fc gamma receptor and does not activate complement (see Muhammed (2020) Immunome Res. 16(1): 173), it does not activate the immune system to the same extent as the IgG1 subclass of antibodies. Consequently, the cytokine expression is stimulated to a lower extent and the risk for a cytokine storm is reduced. The IgG4 subclass of antibodies is able to bind to FcRn.

"A variant of the Fc portion of human IgG4" refers to the Fc portion of human IgG4 which has one or more amino acid substitutions compared to the wild-type Fc portion of human IgG4 according to SEQ ID NO: 3. In one embodiment, the variant of the Fc portion of human IgG4 has one to twelve, one to eleven, one to ten, one to nine, one to eight, one to seven, one to six, one to five, one to four, one to three, one or two amino acid substitutions compared to the wild-type Fc portion of human IgG4 according to SEQ ID NO: 3. In one embodiment, the variant of the Fc portion of human IgG4 has one, two, three, four, five, six, seven, eight, nine, ten, eleven or twelve amino acid substitutions compared to the wild-type Fc portion of human IgG4 according to SEQ ID NO: 3. In one embodiment, the one or more amino acid substitutions lead to decreased effector functions compared to the wild-type Fc portion of human IgG4 according to SEQ ID NO: 3. In one embodiment, the one or more amino acid substitutions lead to an increased half-life compared to the wild-type Fc portion of human IgG4 according to SEQ ID NO: 3. In one embodiment, the one or more amino acid substitutions lead to decreased effector functions compared to the wild-type Fc portion of human IgG4 according to SEQ ID NO: 3 and to an increased half-life compared to the wild-type Fc portion of human IgG4 according to SEQ ID NO: 3.

In one embodiment, the one or more amino acid substitutions do not produce the wild-type Fc portion of IgG1 according to SEQ ID NO: 2. In one embodiment, the one or more amino acid substitutions do not impart upon the altered IgG4 Fc portion the effector functions of wild-type lgG1.

Preferably, the decreased effector functions comprise a decreased complement-dependent cytotoxicity (CDC). More preferably, the CDC is decreased by at least two-fold, at least three-fold, at least four-fold or at least five-fold compared to the CDC of the wild-type Fc portion of human IgG4 according to SEQ ID NO: 3. Methods to determine and quantify CDC are well-known to the skilled person. In general, CDC can be determined by incubating the Fc portion fused to an antigen-binding portion with suitable target cells and complement and detecting the cell death of the target cells. Complement recruitment can be analyzed with a C1q binding assay using ELISA plates (see, e.g., Schlothauer et al. (2016) Protein Eng. Des. Sel. 29(10): 457-466).

In one embodiment, the variant of the Fc portion of human IgG4 comprises at least one amino acid substitution at an amino acid residue selected from F3, L4, G6, P7, F12, V33, N66 and P98 of the sequence according to SEQ ID NO: 3. These amino acid residues correspond to amino acid residues F234, L235, G237, P238, F243, V264, N297 and P329 of full-length human IgG4. It was shown that amino acid substitutions at these residues lead to a reduced effector function (WO 94/28027; WO 94/29351; WO 95/26403; WO 2011/066501; WO 2011/149999; WO 2012/130831; Wang et al. (2018) Protein Cell. 9(1): 63-73).

In one embodiment, the variant of the Fc portion of human IgG4 comprises the amino acid substitution L4E/A in the sequence according to SEQ ID NO: 3 which corresponds to the amino acid substitution L235E/A in the amino acid sequence of full-length human IgG4. This variant has a reduced effector function, in particular reduced CDC.

In one embodiment, the variant of the Fc portion of human IgG4 comprises the amino acid substitutions F3A and L4A in the sequence according to SEQ ID NO: 3 which correspond to the amino acid substitutions F234A and L235A in the amino acid sequence of full-length human IgG4. This variant has a reduced effector function, in particular reduced CDC.

In one embodiment, the variant of the Fc portion of human IgG4 comprises the amino acid substitutions F3A, L4E, G6A and P7S in the sequence according to SEQ ID NO: 3 which correspond to the amino acid substitutions F234A, L235E, G237A and P238S in the amino acid sequence of full-length human IgG4. This variant has a reduced effector function, in particular reduced CDC.

In one embodiment, the variant of the Fc portion of human IgG4 comprises the amino acid substitutions F12A and V33A in the sequence according to SEQ ID NO: 3 which correspond to the amino acid substitutions F243A and V264A in the amino acid sequence of full-length human IgG4. This variant has a reduced effector function, in particular reduced CDC.

In one embodiment, the variant of the Fc portion of human IgG4 comprises the amino acid substitutions L4E and P98G in the sequence according to SEQ ID NO: 3 which correspond to the amino acid substitutions L235E and P329G in the amino acid sequence of full-length human IgG4. This variant has a reduced effector function, in particular reduced CDC.

In one embodiment, the variant of the Fc portion of human IgG4 comprises the amino acid substitution N66A/Q/G in the sequence according to SEQ ID NO: 3 which corresponds to the amino acid substitution N297A/Q/G in the amino acid sequence of full-length human IgG4. This variant has a reduced effector function, in particular reduced CDC.

In one embodiment, the variant of the Fc portion of human IgG4 comprises at least one amino acid substitution at an amino acid residue selected from T250, M252, S254, T256, E258, K288, T307, V308, Q311, V427, M428, H433, N434 and H435 of full-length human IgG4. These amino acid residues correspond to amino acid residues T19, M21, S23, T25, E27, K57, T76, V77, Q80, V196, M197, H202, N203 and H204 of the sequence according to SEQ ID NO: 3. It was shown that these amino acid substitutions lead to an increased half-life of the Fc-containing protein (WO 00/42072; WO 02/060919; WO 2004/035752;
WO 2006/053301; WO 2009/058492; WO 2009/086320; US 2010/0204454; GB 2013/02878; WO 2013/163630; US 2019/0010243). The half-life of an antibody or Fc fusion protein can be determined by measuring the antibody or Fc fusion protein concentration in the serum at different time-points after administration of the antibody or Fc fusion protein and calculating the half-life therefrom.

In one embodiment, the variant of the Fc portion of human IgG4 comprises the amino acid substitutions M21Y, S23T and T25E in the sequence according to SEQ ID NO: 3 which corresponds to the amino acid substitutions M252Y, S254T and T256E in the amino acid sequence of full-length human IgG4. In one embodiment, the variant of the Fc portion of human IgG4 has the amino acid sequence according to SEQ ID NO: 4. This variant has an increased half-life.

In one embodiment, the variant of the Fc portion of human IgG4 comprises the amino acid substitutions T25D and T76Q in the sequence according to SEQ ID NO: 3 which corresponds to the amino acid substitutions T256D and T307Q in the amino acid sequence of full-length human IgG4. In one embodiment, the variant of the Fc portion of human IgG4 has the amino acid sequence according to SEQ ID NO: 5. This variant has an increased half-life and an enhanced binding to FcRn.

In one embodiment, the second part of the fusion protein of the present invention comprises the Fc portion of human IgG1 or a variant thereof. The Fc portion of human IgG1 comprises the CH2 and CH3 domains of human IgG1 linked together to form the Fc portion. In a full-length human IgG1 antibody the Fc portion is connected to the Fab fragment through a hinge region. The Fab fragment comprises the heavy chain variable region and the CH1 domain. Preferably, the Fc portion of human IgG1 used in the fusion protein of the present invention has the sequence according to SEQ ID NO: 2.

"A variant of the Fc portion of human IgG1" refers to the Fc portion of human IgG1 which has one or more amino acid substitutions or deletions compared to the wild-type Fc portion of human IgG1 according to SEQ ID NO: 2. In one embodiment, the one or more amino acid substitutions lead to decreased effector functions compared to the wild-type Fc portion of human IgG1 according to SEQ ID NO: 2.

Preferably, the decreased effector functions comprise a decreased complement-dependent cytotoxicity (CDC). More preferably, the CDC is decreased by at least two-fold, at least three-fold, at least four-fold or at least five-fold compared to the CDC of the wild-type Fc portion of human IgG1 according to SEQ ID NO: 2. Methods to determine and quantify CDC are well-known to the skilled person and have been described above.

In one embodiment, the variant of the Fc portion of human IgG1 comprises at least one amino acid substitution at an amino acid residue selected from L3, L4 and P98 of the sequence according to SEQ ID NO: 2. These amino acid residues correspond to amino acid residues L234, L235, and P329 of full-length human IgG1.

In one embodiment, the variant of the Fc portion of human IgG1 comprises the amino acid substitution L4E/A in the sequence according to SEQ ID NO: 2 which corresponds to the amino acid substitution L235E/A in the amino acid sequence of full-length human IgG1. This variant has a reduced effector function, in particular reduced CDC.

In one embodiment, the variant of the Fc portion of human IgG1 comprises the amino acid substitutions L3A and L4A in the sequence according to SEQ ID NO: 2 which correspond to the amino acid substitutions L234A and L235A in the amino acid sequence of full-length human IgG1. This variant has a reduced effector function, in particular reduced CDC.

In one embodiment, the variant of the Fc portion of human IgG1 comprises the amino acid substitutions L3A, L4A, P98G in the sequence according to SEQ ID NO: 2 which correspond to the amino acid substitutions L234A, L235A and P329G in the amino acid sequence of full-length human IgG1. This variant has a reduced effector function, in particular reduced CDC.

In one embodiment, the variant of the Fc portion of human IgG1 comprises the amino acid substitutions L4A and P98G in the sequence according to SEQ ID NO: 2 which correspond to the amino acid substitutions L235A and P329G in the amino acid sequence of full-length human IgG1. This variant has a reduced effector function, in particular reduced CDC.

In one embodiment, the variant of the Fc portion of human IgG1 comprises the amino acid substitutions M21Y, S23T and T25E in the sequence according to SEQ ID NO: 2 which corresponds to the amino acid substitutions M252Y, S254T and T256E in the amino acid sequence of full-length human IgG1. In one embodiment, the variant of the Fc portion of human IgG1 has the amino acid sequence according to SEQ ID NO: 6. This variant has an increased half-life and an enhanced binding to FcRn.

In one embodiment, the variant of the Fc portion of human IgG1 comprises the amino acid substitutions T25D and T76Q in the sequence according to SEQ ID NO: 2 which corresponds to the amino acid substitutions T256D and T307Q in the amino acid sequence of full-length human IgG1. In one embodiment, the variant of the Fc portion of human IgG1 has the amino acid sequence according to SEQ ID NO: 7. This variant has an increased half-life and an enhanced binding to FcRn.

In one embodiment, the second part of the fusion protein of the present invention comprises the Fc portion of human IgG3 or a variant of the Fc portion of human IgG1, or IgG3 and has reduced binding to FcγRllla compared to a fusion protein comprising the same first part and a second part comprising the Fc portion of wild-type human IgG1. The binding to FcγRllla is decreased by at least two-fold, at least three-fold, at least four-fold, at least five-fold or at least 10-fold compared to the binding of a fusion protein comprising the same first part and a second part comprising the Fc portion of wild-type human IgG1 according to SEQ ID NO: 2.

In one embodiment, the second part of the fusion protein of the present invention comprises the Fc portion of human IgG3 or a variant of the Fc portion of human IgG1, or IgG3 and has reduced binding to FcγRllla and essentially the same binding to FcRn compared to a fusion protein comprising the same first part and a second part comprising the Fc portion of wild-type human IgG1. The term "essentially the same binding to FcRn" means that the binding of the fusion protein comprising the Fc portion of human IgG3 or a variant of the Fc portion of human IgG1 or IgG3 to FcRn differs by not more than 20% or not more than 15%, preferably not more than 10% or not more than 5%, more preferably not more than 3% or not more than 2% and most preferably not more than 1% from the binding of a fusion protein comprising the same first part and a second part comprising the Fc portion of wild-type human IgG1 according to SEQ ID NO: 2.

The binding of fusion proteins to FcγRllla or FcRn can be determined by surface plasmon resonance as described in the examples herein.

In one embodiment, the Fc part of a human antibody is N-glycosylated, i.e. an N-glycan is attached to an asparagine residue of the Fc part of the human antibody. In the context of the Fc part of a human IgG1 antibody, the N-glycans are attached to asparagine 297 in the amino acid sequence of full-length human lgG1, corresponding to asparagine 66 in the amino acid sequence according to SEQ ID NO: 2. In the context of the Fc part of a human IgG4 antibody, the N-glycans are attached to asparagine 297 in the amino acid sequence of full-length human IgG4, corresponding to asparagine 66 in the amino acid sequence according to SEQ ID NO: 3.

According to one aspect of the present invention, 15% to 35%, preferably 16% to 32% and most preferably 17% to 30% of the N-glycans on the Fc part have at least one sialic acid molecule attached to the N-glycan. The sialic acid molecule is attached to the terminal galactose of the N-glycan. In one embodiment, 0% to 5%, preferably 1% to 4% and most preferably 1.5% to 3% of the N-glycans on the Fc part have two sialic acid molecules attached to the N-glycan, meaning that two terminal galactose molecules are linked to a sialic acid molecule.

According to one aspect of the present invention, 15% to 35%, preferably 16% to 32% and most preferably 17% to 30% of the N-glycans on the Fc part of a human IgG1 antibody have at least one sialic acid molecule attached to the N-glycan. The sialic acid molecule is attached to the terminal galactose of the N-glycan. In one embodiment, 0% to 5%, preferably 1% to 4% and most preferably 1.5% to 3% of the N-glycans on the Fc part of a human IgG1 antibody have two sialic acid molecules attached to the N-glycan, meaning that two terminal galactose molecules are linked to a sialic acid molecule.

According to one aspect of the present invention, 15% to 35%, preferably 16% to 32% and most preferably 17% to 30% of the N-glycans on the Fc part of a human IgG4 antibody have at least one sialic acid molecule attached to the N-glycan. The sialic acid molecule is attached to the terminal galactose N-glycan. In one embodiment, 0% to 5%, preferably 1% to 4% and most preferably 1.5% to 3% of the N-glycans on the Fc part of a human IgG4 antibody have two sialic acid molecules attached to the N-glycan, meaning that two terminal galactose molecules are linked to a sialic acid molecule.

It was shown that sialylation of the Fc part of human antibodies prolongs serum half-life of therapeutic antibodies (Wang et al. (2018) Biotechnol. Bioeng. 115(6): 1378-1393).

According to one aspect of the present invention, the Fc portion of a human antibody or fragment or variant thereof is afucosylated. The term "afucosylated" means that a protein, here the Fc portion of a human antibody, comprises a glycan structure lacking a fucose moiety. As shown above, a fucose moiety may be attached to the N-acetylglucosamine moiety within the glycan structure. Afucosylated proteins such as the Fc portion of a human antibody lack this fucose moiety attached to the N-acetylglucosamine moiety. An afucosylated antibody or Fc part of a human antibody has an amount of fucose of 60% or less, 50% or less, 40% or less or 30% or less or 20% or less of the total amount of oligosaccharides at asparagine 297 (which is asparagine 66 in the sequence according to SEQ ID NO: 2 or 3). In one embodiment, the afucosylated antibody or Fc part of a human antibody has an amount of fucose of 40% to 60% of the total amount of oligosaccharides at asparagine 297 (which is asparagine 66 in the sequence according to SEQ ID NO: 2 or 3). In one embodiment, the afucosylated antibody or Fc part of a human antibody does not have any fucose attached to the glycan structure at asparagine 297 (which is asparagine 66 in the sequence according to SEQ ID NO: 2 or 3). In certain embodiments, compositions comprising fusion proteins as described herein contain at least 20%, 30%, 40%, 50%, 60%, 70%, 80%, 90%, 91%, 92%, 93%, 94%, 95%, 96%, 97%, 98%, 99%, or 100% afucosylated molecules. Within the present invention it is not required that all the N-glycosylated fusion proteins are afucosylated.

The afucosylation can be analyzed by different methods known to the skilled person, including digestion of the fusion protein and analysis by peptide mapping. Alternatively, hydrophilic interaction liquid chromatography (HILIC) may be performed for glycoprotein analysis as described in the examples herein.

It was shown that afucosylated antibodies or Fc parts thereof show an increased antibody-dependent cell-mediated cytotoxicity (ADCC) due to a stronger affinity for the FcγRIII (Shields et al. (2002) J. Biol. Chem. 277: 26733-26740), Kanda et al. (2007) J. Biotechnol. 130: 300-310; Yamane-Ohunuki et al. (2004) Biotechnol. Bioeng. 87: 614-622).

Afucosylated fusion proteins can be produced by culturing host cells expressing the fusion protein under specific conditions or by using genetically modified host cells with reduced or abolished fucosylation activity as described further below.

In one embodiment, the first and the second part of the fusion protein of the present invention are linked by a linking sequence. The linking sequence is a short amino acid sequence which does not have a function on its own and which does not affect the folding of the fusion protein. In one embodiment, the linking sequence comprises eight to twenty amino acids, preferably 10 to 18 amino acids, more preferably 11 to 17 amino acids or 12 to 16 amino acids and most preferably 13 amino acids. In one embodiment, if the second part of the fusion protein is the Fc portion of human IgG4 or a variant thereof, the linking sequence comprises eight to twenty amino acids, preferably 10 to 18 amino acids, more preferably 11 to 17 amino acids or 12 to 16 amino acids and most preferably 13 amino acids. In one embodiment, if the second part of the fusion protein is the Fc portion of human IgG1 or a variant thereof, the linking sequence comprises seven to 18 amino acids, preferably 8 to 15 amino acids, more preferably 9 to 14 amino acids or 10 to 13 amino acids and most preferably 11 amino acids.

In one embodiment, the linking sequence consists of small amino acids selected from glycine and serine. An overview of linking sequences is provided in Chen et al. (2013) Adv. Drug Deliv. Rev. 65(10): 1357-1369. In one embodiment, the linking sequence consists of the amino acid sequence according to SEQ ID NO: 8.

In one embodiment, if the second part of the fusion protein is the Fc portion of human IgG4 or a variant thereof, the linking sequence consists of the hinge region of human IgG4. In one embodiment, the linking sequence consists of the sequence according to SEQ ID NO: 11. In the sequence according to SEQ ID NO: 11 the serine at residue 10 of the wild-type hinge region of IgG4 (corresponding to serine 228 of full-length IgG4) has been replaced with proline, leading to a reduction in the exchange of IgG half-molecules. It is known that IgG4 antibodies can undergo Fab-arm exchange, leading to the combination of two distinct Fab arms and creating new bispecific antibody molecules (see, e.g., Aalberse et al. (2009) Clin. Exp. Allergy 39(4): 469-477). This Fab-arm exchange can be prevented by a mutation of serine 228 of the Fc region to proline (S228P; see Silva et al. (2015) J. Biol. Chem. 290: 5462-5469) which is located in the hinge region of IgG4. Further, the use of a short linker sequence increases the stability of the fusion protein and lowers the accessibility of the fusion protein to proteases.

In one embodiment, if the second part of the fusion protein is the Fc portion of human IgG1 or a variant thereof, the linking sequence consists of the hinge region of human IgG1. In one embodiment, the linking sequence consists of the sequence according to SEQ ID NO: 12.

In a particular embodiment, the fusion protein of the present invention has the amino acid sequence according to SEQ ID NO: 27 which comprises a first part consisting of amino acids 18 to 732 of human ACE2 (SEQ ID NO: 1) with mutation T27A, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 28 which comprises a first part consisting of amino acids 18 to 732 of human ACE2 (SEQ ID NO: 1) with mutation T27A, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 29 which comprises a first part consisting of amino acids 18 to 732 of human ACE2 (SEQ ID NO: 1) with mutation T27A, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 30 which comprises a first part consisting of amino acids 18 to 732 of human ACE2 (SEQ ID NO: 1) with mutation T27A, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 31 which comprises a first part consisting of amino acids 18 to 732 of human ACE2 (SEQ ID NO: 1) with mutation T92I, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 32 which comprises a first part consisting of amino acids 18 to 732 of human ACE2 (SEQ ID NO: 1) with mutation T92I, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 33 which comprises a first part consisting of amino acids 18 to 732 of human ACE2 (SEQ ID NO: 1) with mutation T92I, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 34 which comprises a first part consisting of amino acids 18 to 732 of human ACE2 (SEQ ID NO: 1) with mutation T92I, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 35 which comprises a first part consisting of amino acids 18 to 732 of human ACE2 (SEQ ID NO: 1) with mutation Q325P, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 36 which comprises a first part consisting of amino acids 18 to 732 of human ACE2 (SEQ ID NO: 1) with mutation Q325P, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 37 which comprises a first part consisting of amino acids 18 to 732 of human ACE2 (SEQ ID NO: 1) with mutation Q325P, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 38 which comprises a first part consisting of amino acids 18 to 732 of human ACE2 (SEQ ID NO: 1) with mutation Q325P, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 39 which comprises a first part consisting of amino acids 18 to 732 of human ACE2 (SEQ ID NO: 1) with mutations T27A and S645C, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 40 which comprises a first part consisting of amino acids 18 to 732 of human ACE2 (SEQ ID NO: 1) with mutations T27A and S645C, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 41 which comprises a first part consisting of amino acids 18 to 732 of human ACE2 (SEQ ID NO: 1) with mutations T27A and S645C, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 42 which comprises a first part consisting of amino acids 18 to 732 of human ACE2 (SEQ ID NO: 1) with mutations T27A and S645C, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 43 which comprises a first part consisting of amino acids 18 to 732 of human ACE2 (SEQ ID NO: 1) with mutations T92I and S645C, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 44 which comprises a first part consisting of amino acids 18 to 732 of human ACE2 (SEQ ID NO: 1) with mutations T92I and S645C, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 45 which comprises a first part consisting of amino acids 18 to 732 of human ACE2 (SEQ ID NO: 1) with mutations T92I and S645C, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 46 which comprises a first part consisting of amino acids 18 to 732 of human ACE2 (SEQ ID NO: 1) with mutations T92I and S645C, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 47 which comprises a first part consisting of amino acids 18 to 732 of human ACE2 (SEQ ID NO: 1) with mutations Q325P and S645C, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 48 which comprises a first part consisting of amino acids 18 to 732 of human ACE2 (SEQ ID NO: 1) with mutations Q325P and S645C, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 49 which comprises a first part consisting of amino acids 18 to 732 of human ACE2 (SEQ ID NO: 1) with mutations Q325P and S645C, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 50 which comprises a first part consisting of amino acids 18 to 732 of human ACE2 (SEQ ID NO: 1) with mutations Q325P and S645C, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 51 which comprises a first part consisting of amino acids 18 to 732 of human ACE2 (SEQ ID NO: 1) with mutations T27A, T92I and S645C, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 52 which comprises a first part consisting of amino acids 18 to 732 of human ACE2 (SEQ ID NO: 1) with mutations T27A, T92I and S645C, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 53 which comprises a first part consisting of amino acids 18 to 732 of human ACE2 (SEQ ID NO: 1) with mutations T27A, T92I and S645C, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 54 which comprises a first part consisting of amino acids 18 to 732 of human ACE2 (SEQ ID NO: 1) with mutations T27A, T92I and S645C, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 55 which comprises a first part consisting of amino acids 18 to 732 of human ACE2 (SEQ ID NO: 1) with mutations T27A, Q325P and S645C, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 56 which comprises a first part consisting of amino acids 18 to 732 of human ACE2 (SEQ ID NO: 1) with mutations T27A, Q325P and S645C, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 57 which comprises a first part consisting of amino acids 18 to 732 of human ACE2 (SEQ ID NO: 1) with mutations T27A, Q325P and S645C, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 58 which comprises a first part consisting of amino acids 18 to 732 of human ACE2 (SEQ ID NO: 1) with mutations T27A, Q325P and S645C, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 59 which comprises a first part consisting of amino acids 18 to 732 of human ACE2 (SEQ ID NO: 1) with mutations T92I, Q325P and S645C, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 60 which comprises a first part consisting of amino acids 18 to 732 of human ACE2 (SEQ ID NO: 1) with mutations T92I, Q325P and S645C, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 61 which comprises a first part consisting of amino acids 18 to 732 of human ACE2 (SEQ ID NO: 1) with mutations T92I, Q325P and S645C, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 62 which comprises a first part consisting of amino acids 18 to 732 of human ACE2 (SEQ ID NO: 1) with mutations T92I, Q325P and S645C, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 63 which comprises a first part consisting of amino acids 18 to 732 of human ACE2 (SEQ ID NO: 1) with mutations T27A, T92I, Q325P and S645C, the numbering referring to SEQ ID NO: 67, the linking sequence according to SEQ ID NO: 11 and a second part which comprises the variant of the Fc portion of human IgG4 according to SEQ ID NO:4.

In a particular embodiment, the fusion protein of the present invention has the amino acid sequence according to SEQ ID NO: 64 which comprises a first part consisting of amino acids 18 to 732 of human ACE2 (SEQ ID NO: 1) with mutations T27A, T92I, Q325P and S645C, the numbering referring to SEQ ID NO: 67, the linking sequence according to SEQ ID NO: 12 and a second part which comprises the variant of the Fc portion of human IgG1 according to SEQ ID NO:6.

In a particular embodiment, the fusion protein of the present invention has the amino acid sequence according to SEQ ID NO: 65 which comprises a first part consisting of amino acids 18 to 732 of human ACE2 (SEQ ID NO: 1) with mutations T27A, T92I, Q325P and S645C, the numbering referring to SEQ ID NO: 67, the linking sequence according to SEQ ID NO: 11 and a second part which comprises the variant of the Fc portion of human IgG4 according to SEQ ID NO:5.

In a particular embodiment, the fusion protein of the present invention has the amino acid sequence according to SEQ ID NO: 66 which comprises a first part consisting of amino acids 18 to 732 of human ACE2 (SEQ ID NO: 1) with mutations T27A, T92I, Q325P and S645C, the numbering referring to SEQ ID NO: 67, the linking sequence according to SEQ ID NO: 12 and a second part which comprises the variant of the Fc portion of human IgG1 according to SEQ ID NO:7.

In a particular embodiment, the fusion protein of the present invention has the amino acid sequence according to SEQ ID NO: 68 which comprises a first part consisting of amino acids 18 to 732 of human ACE2 (SEQ ID NO: 1) with mutation T27A, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 69 which comprises a first part consisting of amino acids 18 to 732 of human ACE2 (SEQ ID NO: 1) with mutation T27A, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 70 which comprises a first part consisting of amino acids 18 to 732 of human ACE2 (SEQ ID NO: 1) with mutation T92I, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 71 which comprises a first part consisting of amino acids 18 to 732 of human ACE2 (SEQ ID NO: 1) with mutation T92I, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 72 which comprises a first part consisting of amino acids 18 to 732 of human ACE2 (SEQ ID NO: 1) with mutation Q325P, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 73 which comprises a first part consisting of amino acids 18 to 732 of human ACE2 (SEQ ID NO: 1) with mutation Q325P, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 74 which comprises a first part consisting of amino acids 18 to 732 of human ACE2 (SEQ ID NO: 1) with mutations T27A and S645C, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 75 which comprises a first part consisting of amino acids 18 to 732 of human ACE2 (SEQ ID NO: 1) with mutations T27A and S645C, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 76 which comprises a first part consisting of amino acids 18 to 732 of human ACE2 (SEQ ID NO: 1) with mutations T92I and S645C, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 77 which comprises a first part consisting of amino acids 18 to 732 of human ACE2 (SEQ ID NO: 1) with mutations T92I and S645C, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 78 which comprises a first part consisting of amino acids 18 to 732 of human ACE2 (SEQ ID NO: 1) with mutations Q325P and S645C, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 79 which comprises a first part consisting of amino acids 18 to 732 of human ACE2 (SEQ ID NO: 1) with mutations Q325P and S645C, the numbering referring to SEQ ID NO: 67, the linking sequence according to SEQ ID NO: 12 and a second part which comprises the variant of the Fc portion of human IgG1 according to SEQ ID NO:2.

In a particular embodiment, the fusion protein of the present invention has the amino acid sequence according to SEQ ID NO: 80 which comprises a first part consisting of amino acids 18 to 732 of human ACE2 (SEQ ID NO: 1) with mutations T27A, T92I and S645C, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 81 which comprises a first part consisting of amino acids 18 to 732 of human ACE2 (SEQ ID NO: 1) with mutations T27A, T92I and S645C, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 82 which comprises a first part consisting of amino acids 18 to 732 of human ACE2 (SEQ ID NO: 1) with mutations T27A, Q325P and S645C, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 83 which comprises a first part consisting of amino acids 18 to 732 of human ACE2 (SEQ ID NO: 1) with mutations T27A, Q325P and S645C, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 84 which comprises a first part consisting of amino acids 18 to 732 of human ACE2 (SEQ ID NO: 1) with mutations T92I, Q325P and S645C, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 85 which comprises a first part consisting of amino acids 18 to 732 of human ACE2 (SEQ ID NO: 1) with mutations T92I, Q325P and S645C, the numbering referring to SEQ ID NO: 67, the linking sequence according to SEQ ID NO: 12 and a second part which comprises the Fc portion of human IgG1 according to SEQ ID NO:2.

In a particular embodiment, the fusion protein of the present invention has the amino acid sequence according to SEQ ID NO: 86 which comprises a first part consisting of amino acids 18 to 732 of human ACE2 (SEQ ID NO: 1) with mutations T27A, T92I, Q325P and S645C, the numbering referring to SEQ ID NO: 67, the linking sequence according to SEQ ID NO: 11 and a second part which comprises the Fc portion of human IgG4 according to SEQ ID NO:3.

In a particular embodiment, the fusion protein of the present invention has the amino acid sequence according to SEQ ID NO: 87 which comprises a first part consisting of amino acids 18 to 732 of human ACE2 (SEQ ID NO: 1) with mutations T27A, T92I, Q325P and S645C, the numbering referring to SEQ ID NO: 67, the linking sequence according to SEQ ID NO: 12 and a second part which comprises the Fc portion of human IgG1 according to SEQ ID NO:2.

In one embodiment, the fusion protein of the present invention comprises a signal peptide which is located N-terminal of the ACE2 part of the fusion protein. The signal peptide functions to target the protein to the endoplasmic reticulum and ultimately to secretion from the cell. The skilled person knows suitable signal peptides. In one embodiment, the signal peptide is selected from a human albumin signal peptide, a human chymotrypsinogen signal peptide, a human interleukin-2 signal peptide, a human trypsinogen-2 signal peptide or a heavy or light chain signal peptide. In a preferred embodiment, the signal peptide is a human albumin signal peptide. In a particularly preferred embodiment, the signal peptide is a human albumin signal peptide according to SEQ ID NO: 9.

The present invention also relates to a nucleic acid molecule comprising a nucleic acid sequence encoding the fusion protein of the present invention. The skilled person knows how to construct a nucleic acid molecule when the amino acid sequence of a protein is known. In particular, the construction of the nucleic acid molecule involves back-translating the amino acid sequence of the protein into a nucleic acid sequence using the three-letter genetic code and optionally taking into account the codon usage of the host cell in which the protein is to be expressed using the nucleic acid molecule.

In one embodiment, the nucleic acid molecule comprising a nucleic acid sequence encoding the fusion protein of the present invention is an isolated nucleic acid molecule. The term "isolated nucleic acid molecule" refers to a nucleic acid molecule that is identified and separated from at least one contaminant nucleic acid molecule with which it is ordinarily associated in the environment in which it was produced. Preferably, the isolated nucleic acid is free of association with all components associated with the production environment.

The term "vector," as used herein, refers to a nucleic acid molecule capable of propagating another nucleic acid to which it is linked. The term includes the vector as a self- replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

Expression vectors contain elements for the expression of the nucleic acids such as suitable promoters and polyadenylation signals. In addition, the expression vectors typically contain a selection marker gene under the control of suitable promoter to enable the distinction of cells which contain the expression vector from cells which do not contain the expression vector. The elements and methods needed to construct expression vectors which are suitable for expressing a recombinant protein such as the fusion protein of the present invention are well-known to the skilled person and described for example in Makrides et al. (1999) Protein Expr. Purif. 17: 183-202 and Kaufman (2000) Mol. Biotechnol. 16: 151-161.

The expression vector is used to transform, i.e. genetically modify, suitable host cells.
The skilled person is aware of methods for introducing the expression vectors into the mammalian cells. These methods include the use of commercially available transfection kits such as Lipofectamine^{®} of ThermoFisher, PElmax of Polyplus Sciences), 293-Free transfection reagent (Millipore) or Freestyle Max of Invitrogen. Further suitable methods include electroporation, calcium phosphate-mediated transfection and DEAE-dextrane transfection. After transfection the cells are subjected to selection by treatment with a suitable agent based on the selection marker(s) encoded by the expression vector(s) to identify the stably transfected cells which contain the recombinant nucleic acid molecule.

The terms "host cell," "host cell line," and "host cell culture" are used interchangeably and refer to cells into which an exogenous nucleic acid or an expression vector has been introduced, including the progeny of such cells. Host cells include "transformants" and "transformed cells," which include the primary transformed cell and progeny derived therefrom without regard to the number of passages.

The fusion protein of the present invention is preferably produced in mammalian host cells. Suitable mammalian host cells for expressing the fusion protein of the invention include Chinese Hamster Ovary (CHO) cells (including dhfr negative CHO cells used with a DHFR selectable marker), NS0 myeloma cells, COS cells, SP2 cells, monkey kidney CV1, human embryonic kidney line 293, baby hamster kidney cells (BHK), mouse Sertoli cells (TM4), African green monkey kidney cells (VERO-76), human cervical carcinoma cells (HELA), canine kidney cells (MDC), buffalo rat liver cells (BRL 3 A), human lung cells (W138), human liver cells (Hep G2), mouse mammary tumor cells (MMT 060562), TRI cells, MRC 5 cells and FS4 cells. More preferably, the host cells are derived from a rodent. Most preferably, the mammalian cells are Chinese hamster ovary (CHO) cells.

To produce the fusion proteins, the host cells are cultured in a suitable culture medium.

The terms "medium", "cell culture medium" and "culture medium" are interchangeably used herein and refer to a solution containing nutrients which are required for growing mammalian cells. Typically, a cell culture medium provides essential and non-essential amino acids, vitamins, energy sources, lipids, and trace elements required by the cell for minimal growth and/or survival. The cell culture medium may also comprise growth factors. Preferably, the medium is chemically defined in that all its components and their concentration are known. Also preferably, the medium is serum-free and hydrolysate-free and does not contain any components derived from animals. In a more preferred embodiment the medium is serum-free and hydrolysate-free and does not contain any components derived from animals or insulin.

In one embodiment the medium used in the method of the present invention is a commercially available medium such as FreeStyle 293 expression medium (Life Technologies), PolCHO P Powder Base CD, ActiPro (both available from GE), PowerCHO-2, ProCHO-5 (both available from Lonza) or EX-CELL^{®} Advanced CHO fed batch medium (available from Sigma).

For culturing the mammalian cells different strategies are available, including batch culture, perfusion culture, continuous culture and fed-batch culture. Preferably, a fed-batch culture process is used. In fed-batch culture the culturing process is started with a certain volume of the basal medium and one or more feed media comprising one or more nutrients are fed at later time-point(s) of the culture process to prevent nutrient depletion while no product is removed from the cell culture broth. Accordingly, the term "feeding" means that at least one component is added to an existing culture of cells.

The term "basal medium" is intended to refer to the medium which is used from the beginning of the cell culture process. The mammalian cells are inoculated into the basal medium and grown in this medium for a certain period until the feeding is started. The basal medium meets the definition of the culture medium as provided above. If a commercially available medium is used, additional components may be added to the basal medium.

The feed medium is added to the cell culture after the cells have been cultured in the basal medium for a certain period. The feed medium serves to prevent nutrient depletion and therefore may not have the same composition as the basal medium. In particular, the concentration of one or more nutrients may be higher in the feed medium than in the basal medium. In one embodiment, the feed medium has the same composition as the basal medium. In another embodiment, the feed medium has another composition as the basal medium. The feed medium may be added continuously or as a bolus at defined time points.

Suitable feed media are known to the skilled person and include PolCHO Feed-A Powder Base CD, PolCHO Feed-B Powder Base CD, Cell Boost 7a and Cell Boost 7b (all available from GE), BalanCD^{®} CHO Feed 3 Medium (available from Irvine Scientific) and EX-CELL^{®} Advanced CHO feed 1 (available from Sigma).

As discussed above, the sialylation of the fusion protein as described herein can be increased by using specific culturing methods. Hence, in one aspect the present invention relates to a method for producing the fusion protein as described herein with an increased sialylation, comprising culturing a eukaryotic host cell comprising a recombinant nucleic acid molecule encoding said fusion protein in a medium supplemented with one or more of a manganese salt, N-acetylmannosamine or a derivative thereof, galactose, glucosamine or a derivative thereof and DMSO.

It has been shown that the above cell culture additives enhance sialylation of proteins produced in eukaryotic host cells (see, e.g., Liu et al. (2015) World J. Microbiol. Biotechnol. 31(7): 1147-1156; Crowell et al. (2007) Biotechnol. Bioeng. 96(3): 538-549; Lee et al. (2017) Biotechnol. Bioeng. 114(9): 1991-2000); WO 2011/061275; WO 2004/008100).

In one embodiment, the method comprises culturing the eukaryotic host cell comprising a recombinant nucleic acid molecule encoding said fusion protein in a medium supplemented with 20 to 80 µM of a manganese salt, preferably with 25 to 70 µM of a manganese salt, more preferably with 30 µM to 60 µM of a manganese salt and most preferably with 40 µM of a manganese salt. In one embodiment, the method comprises culturing the eukaryotic host cell comprising a recombinant nucleic acid molecule encoding said fusion protein in a medium supplemented with 20 to 80 µM of manganese chloride, preferably with 25 to 70 µM of manganese chloride, more preferably with 30 µM to 60 µM of manganese chloride and most preferably with 40 µM of manganese chloride.

In one embodiment, the method comprises culturing the eukaryotic host cell comprising a recombinant nucleic acid molecule encoding said fusion protein in a medium supplemented with 5 to 30 mM N-acetylmannosamine, preferably with 6 to 25 mM N-acetylmannosamine, more preferably with 8 to 20 mM N-acetylmannosamine and most preferably with 10 mM N-acetylmannosamine.

In one embodiment, the method comprises culturing the eukaryotic host cell comprising a recombinant nucleic acid molecule encoding said fusion protein in a medium supplemented with 20 to 80 µM of a manganese salt and 5 to 30 mM N-acetylmannosamine, preferably with 25 to 70 µM of a manganese salt and 6 to 25 mM N-acetylmannosamine, more preferably with 30 µM to 60 µM of a manganese salt and 8 to 20 mM N-acetylmannosamine and most preferably with 40 µM of a manganese salt and 10 mM N-acetylmannosamine. In one embodiment, the method comprises culturing the eukaryotic host cell comprising a recombinant nucleic acid molecule encoding said fusion protein in a medium supplemented with 20 to 80 µM of manganese chloride and 5 to 30 mM N-acetylmannosamine, preferably with 25 to 70 µM of manganese chloride and 6 to 25 mM N-acetylmannosamine, more preferably with 30 µM to 60 µM of manganese chloride and 8 to 20 mM N-acetylmannosamine and most preferably with 40 µM of manganese chloride and 10 mM N-acetylmannosamine.

In one embodiment, the method comprises culturing the eukaryotic host cell comprising a recombinant nucleic acid molecule encoding said fusion protein in fed-batch mode as discussed above.

In the fed-batch mode, the eukaryotic host cell is preferably fed with a manganese salt and/or galactose, preferably with manganese chloride and/or galactose.

In one embodiment, the eukaryotic host cell is fed with 20 to 80 µM of a manganese salt, preferably with 25 to 70 µM of a manganese salt, more preferably with 30 µM to 60 µM of a manganese salt and most preferably with 40 µM of a manganese salt. In one embodiment, the eukaryotic host cell is fed with 20 to 80 µM of manganese chloride, preferably with 25 to 70 µM of manganese chloride, more preferably with 30 µM to 60 µM of manganese chloride and most preferably with 40 µM of manganese chloride.

In one embodiment, the eukaryotic host cell is fed with a composition comprising amino acids, vitamins, salts, trace elements, glucose and 20 to 80 µM of a manganese salt, preferably 25 to 70 µM of a manganese salt, more preferably 30 µM to 60 µM of a manganese salt and most preferably 40 µM of a manganese salt. In one embodiment, the eukaryotic host cell is fed with a composition comprising amino acids, vitamins, salts, trace elements, glucose and 20 to 80 µM of manganese chloride, preferably 25 to 70 µM of manganese chloride, more preferably 30 µM to 60 µM of manganese chloride and most preferably with 40 µM of manganese chloride.

In one embodiment, the eukaryotic host cell is fed with 100 mM to 250 mM galactose, preferably with 120 mM to 230 mM galactose, more preferably with 150 mM to 200 mM galactose and most preferably with 185 mM galactose.

In one embodiment, the eukaryotic host cell is fed with 20 to 80 µM of a manganese salt and 100 mM to 250 mM galactose, preferably with 25 to 70 µM of a manganese salt and 120 mM to 230 mMgalactose, more preferably with 30 µM to 60 µM of a manganese salt and 150 mM to 200 mM galactose and most preferably with 40 µM of a manganese salt and 185 mM galactose. In one embodiment, the eukaryotic host cell is fed with 20 to 80 µM of manganese chloride and 100 mM to 250 mM galactose, preferably with 25 to 70 µM of manganese chloride and 120 mM to 230 mM galactose, more preferably with 30 µM to 60 µM of manganese chloride and 150 mM to 200 mM galactose and most preferably with 40 µM of manganese chloride and 185 mM galactose.

In one embodiment, the eukaryotic host cell is fed with a composition comprising amino acids, vitamins, salts, trace elements, glucose, 20 to 80 µM of a manganese salt and 100 mM to 250 mM galactose, preferably with a composition comprising amino acids, vitamins, salts, trace elements, glucose, 25 to 70 µM of a manganese salt and 120 mM to 230 mMgalactose, more preferably with a composition comprising amino acids, vitamins, salts, trace elements, glucose, 30 µM to 60 µM of a manganese salt and 150 mM to 200 mM galactose and most preferably with a composition comprising amino acids, vitamins, salts, trace elements, glucose, 40 µM of a manganese salt and 185 mM galactose. In one embodiment, the eukaryotic host cell is fed with a composition comprising amino acids, vitamins, salts, trace elements, glucose, 20 to 80 µM of manganese chloride and 100 mM to 250 mM galactose, preferably with a composition comprising amino acids, vitamins, salts, trace elements, glucose, 25 to 70 µM of manganese chloride and 120 mM to 230 mM galactose, more preferably with a composition comprising amino acids, vitamins, salts, trace elements, glucose, 30 µM to 60 µM of manganese chloride and 150 mM to 200 mM galactose and most preferably with a composition comprising amino acids, vitamins, salts, trace elements, glucose, 40 µM of manganese chloride and 185 mM galactose.

As discussed above, afucosylated fusion proteins can be produced by using specific culturing methods. Hence, in one aspect the present invention relates to a method for producing an afucosylated fusion protein as described herein, comprising culturing a eukaryotic host cell comprising a recombinant nucleic acid molecule encoding said fusion protein in a medium supplemented with a fucosylation inhibitor.

A "fucosylation inhibitor" is a molecule which binds to an enzyme involved in protein fucosylation, in particular to a fucosyltransferase, and inhibits its activity. Suitable fucosylation inhibitors include, but are not limited to, 2-fluorofucose, A2FF1P, B2FF1P (Pijnenborg et al. (2020) Chem. Eur. J. 27: 4022-4027), 2F-peracetyl-fucose, peracetylated 5-thiofucose, 2-deoxy-D-galactose, peracetylated 6-alkyyl-fucose and 6,6,6-trifluorofucose (Li et al. (2018) Cell Chemical Biology 25: 499-512), GDP-D-rhamnose, Ac-GDP-D-rhamnose, sodium rhamnose phosphate (WO 2021/127414), 5-alkynyl-fucose and 5-alkynyl-fucose peracetate (Zimermann et al. (2019) Antibodies 8(1): 9).

Preferably, the fucosylation inhibitor is 2-fluorofucose. Preferably, the 2-fluorofucose is added at a concentration of 5 to 200 µM, preferably at a concentration of 10 to 150 µM.

In another aspect, the present invention relates to a method for producing an afucosylated fusion protein as described herein, comprising culturing a eukaryotic host cell comprising a recombinant nucleic acid molecule encoding said fusion protein, wherein the eukaryotic host cell is genetically modified to decrease the activity of a protein involved in fucosylation.

In one embodiment, the protein involved in fucosylation is selected from the group consisting of GDP-mannose 4,6-dehydratase (GMD) (Kanda et al. (2007) J. Biotechnol. 130: 300-310), GDP-fucose transporter (GFT) (Bardhi et al. (2017) J. Virol. 91(20): e937-917; Chen et al. (2016) MABs 8: 761-774) and alpha-(1 ,6)-fucosyltransferase (FUT8) (Mori et al. (2004) Biotechnol. Bioeng. 88: 901-908; Imai-Nishiya et al. (2007) BMC Biotechnol. 7:84; Dicker and Strasser (2015) Expert Opin. Bio. Ther. 15: 1501-1516).

The culturing of the host cell may be performed at a constant temperature, e.g. at a temperature of 37°C± 0.2°C. Alternatively, the culture temperature may be reduced from a first temperature to a second temperature, i.e. the temperature is actively downregulated. Hence, the second temperature is lower than the first temperature. The first temperature may be 37°C ± 0.2°C. The second temperature may be in the range of from 30°C to 36°C.

After the fusion protein of the present invention has been produced by culturing the host cell in a suitable culture medium, the fusion protein is harvested from the cell culture. Since Fc fusion proteins expressed from mammalian cells are typically secreted into the cell culture fluid during the cultivation process, the product harvest at the end of the cultivation process occurs by separating cell culture fluid comprising the fusion protein from the cells. The cell separation method should be gentle to minimize cell disruption to avoid the increase of cell debris and release of proteases and other molecules that could affect the quality of the fusion protein product. Usually, the harvesting of the cell culture fluid comprising the fusion protein involves centrifugation and/or filtration, whereby the fusion protein is present in the supernatant and the filtrate, respectively. Expanded bed adsorption chromatography is an alternative method to avoid centrifugation/filtration methods.

After harvesting the cell culture fluid comprising the fusion protein, the fusion protein has to be purified from the cell culture fluid. The purification of Fc fusion proteins is usually accomplished by a series of standard techniques that can include chromatographic steps such as anion exchange chromatography, cation exchange chromatography, affinity chromatography and in particular protein A affinity chromatography, hydrophobic interaction chromatography, hydroxyapatite chromatography and size exclusion chromatography. Further, the purification process may comprise one or more ultra-, nano- or diafiltration as well as tangential flow filtration and/or cross flow filtration steps.

After purifying the fusion protein it can be used to prepare a pharmaceutical composition. A pharmaceutical composition is a composition which is intended to be delivered to a patient for treating or preventing a disease or condition. In addition to the active agent, i.e. the fusion protein of the present invention, a pharmaceutical composition typically contains at least one pharmaceutically acceptable excipient. Pharmaceutically acceptable excipients are substances which do not interfere with the physiological activity of the fusion protein and which stabilize the pharmaceutical composition and/or enhance solubility or decrease viscosity of the pharmaceutical composition. Typical pharmaceutically acceptable excipients for recombinant proteins include buffers, salts, sugars or sugar alcohols, amino acids and surface-active agents.

The pharmaceutical composition comprises a therapeutically effective amount of the fusion protein of the present invention. The term "therapeutically effective amount" refers to an amount of the fusion protein of the present invention sufficient to treat a specified disorder, condition or disease such as ameliorate, palliate, lessen, and/or delay one or more of its symptoms. With respect to an infection with a coronavirus and in particular SARS-CoV-2 the therapeutically effective amount of the fusion protein of the present invention ameliorates, palliates, lessens, and/or delays one or more of symptoms selected from coughing, shortness of breath, difficulty breathing, fever, chills, tiredness, muscle aches, sore throat, headache, chest pain and loss of smell and/or taste. A therapeutically effective amount can be administered in one or more administrations.

The fusion protein of the present invention is for medical use, i.e. it is intended to be used to prevent and/or treat a disease.

As used herein, "treatment" or"treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread of the disease, preventing or delaying the recurrence of the disease, delaying or slowing the progression of the disease, ameliorating the disease state, providing a remission (partial or total) of the disease, decreasing the dose of one or more other medications required to treat the disease, and/or prolonging survival. The use of the present invention contemplates any one or more of these aspects of treatment.

The term "prevent," and similar words such as "prevented", "preventing" etc., indicate an approach for preventing, inhibiting, or reducing the likelihood of the recurrence of, a disease or condition. It also refers to delaying the recurrence of a disease or condition or delaying the recurrence of the symptoms of a disease or condition. As used herein, "prevention" and similar terms also includes reducing the intensity, effect, symptoms and/or burden of a disease or condition prior to recurrence of the disease or condition.

In one embodiment, the fusion protein of the present invention is used to prevent and/or treat an infection with a coronavirus binding to ACE2. Coronaviruses are enveloped viruses with a positive sense, single-stranded RNA genome and an icosahedral protein shell. The Spike protein consisting of the S1 and S2 subunits forms a homotrimer which projects from the envelope and mediates the interaction with the target cells by binding to ACE2. Coronaviruses often cause respiratory diseases in humans and other mammalian as well as bird species. In humans, seven coronavirus strains are known: HCoV-OC43, HCoV-HKU1, HCoV-229E, HCoV-NL63, MERS-CoV, SARS-CoV and SARS-CoV-2. The first four coronavirus strains (HCoV-OC43, HCoV-HKU1, HCoV-229E, HCoV-NL63) cause only mild symptoms, whereas infection with MERS-CoV, SARS-CoV and SARS-CoV-2 may lead to severe, potentially life-threatening disease.

It has been shown that SARS-CoV, SARS-CoV-2 and HCoV-NL63 bind to ACE2 and use this binding to enter the target cells (Li et al. (2003) Nature 426(6965): 450-4; Hoffmann et al. (2020) Cell 181: 1-10; Hofmann et al. (2005) Proc Natl Acad Sci USA. 102(22):7988-93). Accordingly, the fusion protein of the present invention can be used to treat and/or prevent infection with a coronavirus binding to ACE2, in particular infection with SARS-CoV, SARS-CoV-2 or HCoV-NL63. Further coronaviruses binding to ACE2 can be identified by inoculating cells expressing ACE2 either transiently or constitutively with pseudotyped VSV (vesicular stomatitis virus) expressing the coronavirus Spike protein and a reporter protein and detecting the activity of the reporter protein after the inoculation period (see protocol in Hoffmann et al. (2020) Cell 181: 1-10). In one embodiment, the fusion protein of the present invention is used to treat and/or prevent infection with a coronavirus binding to ACE2, wherein the coronavirus binding to ACE2 is not SARS-CoV.

In one embodiment, the fusion protein of the present invention is used to treat and/or prevent infection with a coronavirus binding to ACE2, wherein the coronavirus binding to ACE2 is SARS-CoV-2 or a variant of SARS-CoV-2 comprising the amino acid substitution D614G and/or the amino acid substitution N439K. The variant of SARS-CoV-2 comprising the amino acid substitution D614G is described in Korber et al. (2020) Cell 182(4): 812-827 and the amino acid substitution N439K is described in Thomson et al. (2021) Cell 184(5): 1171,1187.e20; available at https://doi.org/10.1101/2020.11.04.355842. In one embodiment, the fusion protein of the present invention is used to treat and/or prevent infection with a coronavirus binding to ACE2, wherein the coronavirus binding to ACE2 is a variant of SARS-CoV-2 comprising the amino acid substitution D614G. The amino acid substitution D614G is caused by an A-to-G nucleotide mutation at position 23,403 in the Wuhan reference strain. The numbering of the amino acids in the variants refers to the numbering in the Spike protein of SARS-CoV-2 according to SEQ ID NO: 10. Hence, a SARS-CoV-2 virus with the Spike protein according to SEQ ID NO: 10 is defined to be the wild-type SARS-CoV-2 from which any variants are derived.

In one embodiment, the fusion protein of the present invention is used to treat and/or prevent infection with a coronavirus binding to ACE2, wherein the coronavirus binding to ACE2 is a variant of SARS-CoV-2 comprising the amino acid substitution D614G and at least one additional amino acid substitution. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, N501Y, A570D, P681H, T716I, S982A and D1118H and comprising a deletion of amino acids 69, 70 and 145. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, Y453F, I692V and M1229I and comprising a deletion of amino acids 69 and 70. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, S13I, W152C and L452R. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, E484K and V1176F. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, L18F, T20N, P26S, D138Y, R190S, K417T, E484K, N501Y, H655Y, T1027I and V1176F. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, D80A, D215G, K417N, E484K, N501Y and A701V and comprising a deletion of amino acids 242, 243 and 244. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, L18F, D80A, D215G, K417N, E484K, N501Y and A701V and comprising a deletion of amino acids 242, 243 and 244. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, D80A, R246I, K417N, E484K, N501Y and A701V and comprising a deletion of amino acids 242, 243 and 244. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions E484Q and L452R. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions E484K and D614G and comprising a deletion of amino acids 145 and 146.

The numbering of the amino acids in the variants refers to the numbering in the Spike protein of SARS-CoV-2 according to SEQ ID NO: 10. For the purposes of defining variants, the amino acid sequence according to SEQ ID NO: 10 is considered to be the wild-type sequence of the Spike protein of SARS-CoV-2.

In one embodiment, the fusion protein of the present invention is used to treat and/or prevent infection with a coronavirus binding to ACE2, wherein the coronavirus binding to ACE2 is a variant of SARS-CoV-2 comprising one or more amino acid substitutions in the receptor-binding domain of the Spike protein of SARS-CoV-2. The receptor-binding domain of the Spike protein of SARS-CoV-2 comprises amino acids 331 to 524 of SEQ ID NO: 10 (see Tai et al. (2020) Cell. Mol. Immunol. 17: 613-620). In one embodiment, the fusion protein of the present invention is used to treat and/or prevent infection with a coronavirus binding to ACE2, wherein the coronavirus binding to ACE2 is a variant of SARS-CoV-2 comprising the amino acid substitution N501Y. In one embodiment, the fusion protein of the present invention is used to treat and/or prevent infection with a coronavirus binding to ACE2, wherein the coronavirus binding to ACE2 is a variant of SARS-CoV-2 comprising the amino acid substitution E484K. In one embodiment, the fusion protein of the present invention is used to treat and/or prevent infection with a coronavirus binding to ACE2, wherein the coronavirus binding to ACE2 is a variant of SARS-CoV-2 comprising the amino acid substitution K417T/N.

In one embodiment, the fusion protein of the present invention is used to treat and/or prevent infection with a coronavirus binding to ACE2, wherein the coronavirus binding to ACE2 is a variant of SARS-CoV-2 which has a higher binding affinity to ACE2 compared to the SARS-CoV-2 comprising the wild-type Spike protein according to SEQ ID NO: 10. The affinity of a variant of SARS-CoV-2 to ACE2 can for example be determined using a pseudovirus assay. The pseudovirus assay uses a lentivirus which is pseudotyped with the S protein of wild-type SARS-CoV-2 or of a variant thereof and which contains a reporter gene such as the luciferase gene. Such lentiviruses can be obtained for example from BPS Bioscience. The pseudotyped lentivirus is incubated with ACE2 expressing cells to allow the virus to enter the cells and to express the reporter gene. If the expression of the reporter gene from a lentivirus pseudotyped with the S protein of a variant SARS-CoV-2 is higher than the expression of the reporter gene from a lentivirus pseudotyped with the S protein of wild-type SARS-CoV-2, the variant has a higher binding affinity to ACE2. In the context of the present invention it has been found that the fusion proteins of the present invention have a higher affinity to those variants which have a higher binding affinity to ACE2, such as the variant B.1.1.7.

In one embodiment, the fusion protein of the present invention is used to treat and/or prevent infection with a coronavirus binding to ACE2, wherein the coronavirus binding to ACE2 is a variant of SARS-CoV-2 which has a higher transmissibility compared to the SARS-CoV-2 comprising the wild-type Spike protein according to SEQ ID NO: 10. The viral transmissibility can be determined using the basic reproduction number R₀ which is the average number of people who will catch a disease from one contagious person.

The route of administration is in accordance with known and accepted methods, e.g., injection or infusion by subcutaneous, intravenous, intraperitoneal, intramuscular, intraarterial, intralesional or intraarticular routes. In another embodiment the fusion protein of the present invention is to be administered intranasally, e.g. by means of a nasal spray, a nasal ointment or nasal drops. In another embodiment, the fusion protein of the present invention is administered by topical administration or by inhalation. Preferably, the fusion protein of the present invention is administered by intravenous injection or infusion.

Dosages and desired drug concentrations of pharmaceutical compositions of the present invention may vary depending on the particular use envisioned. The determination of the appropriate dosage or route of administration is well within the skill of an ordinary artisan. Animal experiments provide reliable guidance for the determination of effective doses for human therapy. Interspecies scaling of effective doses can be performed following the principles laid down by Mordenti, J. and Chappell, W. "The Use of Interspecies Scaling in Toxicokinetics," In Toxicokinetics and New Drug Development, Yacobi et al., Eds, Pergamon Press, New York 1989, pp.42-46.

In one embodiment, the fusion protein of the present invention is administered intravenously at a dosage of 2 mg/kg body weight to 10 mg/kg body weight, such as a dosage of 2 mg/kg body weight, 2.5 mg/kg body weight, 3.0 mg/kg body weight, 3.5 mg/kg body weight, 4.0 mg/kg body weight, 4.5 mg/kg body weight, 5.0 mg/kg body weight, 5.5 mg/kg body weight, 6.0 mg/kg body weight, 6.5 mg/kg body weight, 7.0 mg/kg body weight, 7.5 mg/kg body weight, 8.0 mg/kg body weight, 8.5 mg/kg body weight, 9.0 mg/kg body weight, 9.5 mg/kg body weight or 10 mg/kg body weight.

In one embodiment, the fusion protein of the present invention is administered intravenously at a dosage of 0.1 mg/kg body weight to 4 mg/kg body weight, such as a dosage of 0.1 mg/kg body weight, 0.2 mg/kg body weight, 0.3 mg/kg body weight, 0.4 mg/kg body weight, 0.5 mg/kg body weight, 0.6 mg/kg body weight, 0.7 mg/kg body weight, 0.8 mg/kg body weight, 0.9 mg/kg body weight, 1.0 mg/kg body weight, 1.1 mg/kg body weight, 1.2 mg/kg body weight, 1.3 mg/kg body weight, 1.4 mg/kg body weight, 1.5 mg/kg body weight, 1.6 mg/kg body weight, 1.7 mg/kg body weight, 1.8 mg/kg body weight, 1.9 mg/kg body weight, 2.0 mg/kg body weight, 2.1 mg/kg body weight, 2.2 mg/kg body weight, 2.3 mg/kg body weight, 2.4 mg/kg body weight, 2.5 mg/kg body weight, 2.6 mg/kg body weight, 2.7 mg/kg body weight, 2.8 mg/kg body weight, 2.9 mg/kg body weight, 3.0 mg/kg body weight, 3.1 mg/kg body weight, 3.2 mg/kg body weight, 3.3 mg/kg body weight, 3.4 mg/kg body weight, 3.5 mg/kg body weight, 3.6 mg/kg body weight, 3.7 mg/kg body weight, 3.8 mg/kg body weight, 3.9 mg/kg body weight or 4.0 mg/kg body weight.

In one embodiment, the fusion protein of the present invention is administered intravenously at a dosage of 10 mg/kg body weight to 150 mg/kg body weight, such as a dosage of 10 mg/kg body weight, 15 mg/kg body weight, 20 mg/kg body weight, 25 mg/kg body weight, 30 mg/kg body weight, 35 mg/kg body weight, 40 mg/kg body weight, 45 mg/kg body weight, 50 mg/kg body weight, 55 mg/kg body weight, 60 mg/kg body weight, 65 mg/kg body weight, 70 mg/kg body weight, 75 mg/kg body weight, 80 mg/kg body weight, 85 mg/kg body weight, 90 mg/kg body weight, 95 mg/kg body weight, 100 mg/kg body weight, 105 mg/kg body weight, 110 mg/kg body weight, 115 mg/kg body weight, 120 mg/kg body weight, 125 mg/kg body weight, 130 mg/kg body weight, 135 mg/kg body weight, 140 mg/kg body weight, 145 mg/kg body weight or 150 mg/kg body weight.

The fusion protein may be administered once per day, twice per day, three times per day, every other day, once per week, once every two weeks or once per month.

The fusion protein may be administered for a period of three days, four days, five days, six days, seven days, eight days, nine days or ten days.

By administering the fusion protein of the present invention, the infection with a coronavirus and in particular with SARS-CoV-2 is treated, i.e. at least one of the symptoms of an infection with SARS-CoV-2 is reduced or abolished. Symptoms of an infection with SARS-CoV-2 include coughing, shortness of breath, difficulty breathing, fever, chills, tiredness, muscle aches, sore throat, headache, chest pain and loss of smell and/or taste. In one embodiment, by the administration of the fusion protein of the present invention the fever caused by infection with SARS-CoV-2 is reduced. In one embodiment, the administration of the fusion protein of the present invention to a subject reduces the risk that the subject experiences a severe course of infection with SARS-CoV-2. In one embodiment, the administration of the fusion protein of the present invention to a subject reduces the risk that the subject experiences multi-organ failure, acute respiratory distress syndrome (ARDS) or pneumonia. In one embodiment, the administration of the fusion protein of the present invention to a subject reduces the risk that the subject experiences long-term effects of the infection with SARS-CoV-2 such as lung damage, neurological disorders, dermatological disorders and cardiovascular disease. In one embodiment, the administration of the fusion protein of the present invention to a subject reduces the concentration of the cytokines IL6 and/or IL8 in the blood. In one embodiment, the administration of the fusion protein of the present invention to a subject reduces the concentration of SARS-CoV-2 virus particles in the blood. In one embodiment, the administration of the fusion protein of the present invention to a subject stimulates the production of antiviral antibodies. In one embodiment, the administration of the fusion protein of the present invention to a subject stimulates the production of antiviral IgA and/or IgG antibodies.

In one embodiment, the fusion protein of the present invention is administered to a subject suffering from a severe infection with SARS-CoV-2. In one embodiment, the fusion protein of the present invention is administered to a subject infected with SARS-CoV-2 and requiring artificial ventilation. In one embodiment, the fusion protein of the present invention is administered to a subject infected with SARS-CoV-2 and requiring extracorporeal membrane oxygenation (ECMO).

By administering the fusion protein of the present invention, the infection with a coronavirus and in particular with SARS-CoV-2 is prevented, i.e. the treated subject does not develop symptoms of an infection with SARS-CoV-2.

In one embodiment, the fusion protein of the present invention is administered to a subject which has been in contact with a subject infected with SARS-CoV-2. Subjects which have been in contact with a subject infected with SARS-CoV-2 can be identified by use of a "Corona warning app" installed on the smartphone.

In one embodiment, the fusion protein of the present invention is administered to a subject for which a test with a throat or nasal swab of said subject indicates that it is infected with SARS-CoV-2, but which has not developed any symptoms of an infection with SARS-CoV-2.

In the treatment or prevention of an infection with a coronavirus binding to ACE2 and in particular SARS-CoV-2 the fusion protein of the present invention may be combined with a known anti-viral agent. Anti-viral agents are medicaments used to treat viral infections and include both specific anti-viral agents and broad-spectrum viral agents. Suitable anti-viral agents include, but are not limited to, nucleoside analoga, inhibitors of viral protease, inhibitors of viral polymerase, blockers of virus entry into the cell, Janus kinase inhibitors, but also inhibitors of inflammatory mediators.

In specific embodiments, the anti-viral agent is selected from the group consisting of remdesivir, arbidol HCl, ritonavir, nirmatrelvir, lopinavir, darunavir, molnupiravir, ribavirin, chloroquin and derivatives thereof such as hydroxychloroquin, nitazoxanide, camostat mesilate, anti-IL6 and anti-IL6 receptor antibodies such as tocilizumab, siltuximab and sarilumab and baricitinib phosphate and combinations of the aforementioned.

In the treatment or prevention of an infection with SARS-CoV-2 the fusion protein of the present invention may be combined with an anti-SARS-CoV-2 monoclonal antibody. Anti-SARS-CoV2 monoclonal antibodies include, but are not limited to, bamlanivimab (LY-CoV555 20; LY3819253;) developed by Eli Lilly and Company, etesevimab (LY-3832479; LY-COV016; JS-016; NP-005), REGN-COV2 which is a cocktail of REGN10933 (casivirimab) and REGN10987 (imdevimab) and which is developed by Regeneron, sotrovimab (VIR-7831; GSK4182136;) developed by Vir Biotechnology and GlaxoSmithKline, CT-P59 developed by Celltrion, AZD 7442 which is a combination of antibodies AZD8895 and AZD1061 and which is developed by Astra Zeneca, JS016 developed by Junshi Biosciences, TY027 developed 25 by Tychan Pte Ltd, BRII-96 and BRII-98 developed by Brii Biosciences, SCTA01 developed by Sinocelltech Ltd, ADM03820 developed by Ology Bioservices, BI767551 developed by Boehringer Ingelheim, bebtelovimab developed by Eli Lilly and AbCellera, a combination of tixagevimab and cilgavimab developed by AstraZeneca AB and COR-101 developed by Corat Therapeutics and others.

Apart from its function in binding coronaviruses, ACE2 has also been implicated in several disorders and diseases such as hypertension (including high blood pressure), congestive heart failure, chronic heart failure, acute heart failure, contractile heart failure, myocardial infarction, arteriosclerosis, kidney failure, renal failure, Acute Respiratory Distress Syndrome (ARDS), Acute Lung Injury (ALI), chronic obstructive pulmonary disease (COPD), pulmonary hypertension, renal fibrosis, chronic renal failure, acute renal failure, acute kidney injury, inflammatory bowel disease and multi-organ dysfunction syndrome. Hence, the fusion protein of the present invention can also be used in the treatment of these disorders and diseases.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

### EXAMPLES

### 1. Construction of fusion proteins

Fusion proteins of the present invention were constructed according to the following Table 1.

**Table 1: Parts of the fusion proteins tested**

| Fusion protein no. | ACE2 | Mutation(s) | Linker | Fc portion | SEQ ID No. |
|---|---|---|---|---|---|
| 1 | 18-732 | T92I | IgG1 | IgG1 YTE | 32 |
| 2 | 18-732 | T92I, S645C | IgG1 | IgG1 YTE | 44 |
| 3 | 18-732 | S645C | IgG1 | IgG1 YTE | n.a. |
| 4 | 18-732 | none | IgG1 | IgG1 YTE | n.a. |
| 5 | 18-732 | S645C | IgG1 | IgG1 | n.a. |
| 6 | 18-732 | T92I | IgG1 | IgG1 | 71 |
| 7 | 18-732 | T92I, S645C | IgG1 | IgG1 | 77 |

The nucleic acid sequence encoding the construct was inserted into an expression vector which was then used to generate stable CHO cell lines expressing the protein of interest. The fusion proteins produced in CHO cells were purified using protein A and size exclusion chromatography and transferred into a buffer.

### 2. Determination of binding of the fusion proteins to the spike protein of SARS-CoV2

### Surface plasmon resonance:

The binding of the fusion proteins to the spike protein of SARS-CoV2 was analyzed by surface plasmon resonance (Biacore) using commercially available SARS-CoV2 spike protein (ACROBiosystems, Newark, USA).

### Binding ELISA:

### Materials

| **Product Name** | **Supplier/vendor** |
|---|---|
| 96 well plate (NUNC) | Thermo Scientific |
| Coating: 2019-nCoV S-protein | Acro Biosystems |
| Detection antibody: GaHu IgG, Fcy fragment - HRP | Jackson ImmunoResearch |
| Wash buffer 20× stock | Pierce |

| | |
|---|---|
| *Wash buffer 1*× = *10 mM sodium phosphate, 0.15 M NaCl, 0.05% Tween-20, pH 7.5* | |

### Analysis

- Coat 96 well plate (NUNC).
- Next day remove coating and wash plate 3× (300 µL/well) with wash buffer
- Block wells with 200 µL/well block buffer (wash buffer supplemented with 1% BSA) and incubate for 1 h at RT and 150 rpm
- Remove block buffer (flick off plate)
- Apply 100 µL/well sample (preparative duplicates) and incubate for 1 h at RT and 150 rpm (Concentration range: 2500 - 833.3 - 277.8 - 92.6 - 30.9 - 10.3 - 3.4 - 1.1 - 0.38 - 0.13 - 0.04 - 0 ng/mL)
- Wash plate 3× (300 µL/well) with wash buffer
- Apply detection Ab (diluted 1/5000 in block buffer) and incubate for 1 h at RT and 150 rpm
- Wash plate 3× (300 µL/well) with wash buffer
- Apply 100 µl of TMB solution (Invitrogen SB02, prewarmed at RT) per well and incubate at RT for 2 min
- Stop reaction with 1 M HCl
- Incubate for 15 min at RT and protected from light
- Measure OD 450 nm and reference at OD 655 nm using a microplate reader (Synergy HTX, BioTek)
- Plot ACE2-Fc concentrations (in µg/mL) on x-axis against OD 450 nm (after subtraction of background values = no ACE2-Fc) on y-axis using a 4-parameter logistic curve fit model

### 3. Determination of binding affinity of the ACE2 fusion proteins to Fc-receptors using surface plasmon resonance (SPR) and the Lumit assay

A Biacore T200 was used for the Fc-receptor binding studies. For the FcγRI and the FcγRIIIa experiments, the His-tagged FcγRI and FcγRIIIa with a concentration of 1.5 nM were captured by injection of the solutions for 90 seconds with a flow rate of 5 µL/min over a covalently immobilized anti-his tag antibody on a CM5 chip. The running buffer was HBS-EP+ pH 7.4 (Cytiva). Five different concentrations of the ACE2-Fc constructs were injected in a single-cycle kinetic mode (3.7-300 nM for the experiment with FcγRI and 25-2000 nM for FcγRIIIa). The FcγRI-binding data was fit to a heterogeneous ligand model and the first binding constant was reported. The FcγRIIIa-binding data was fit to a two-state reaction model to derive the binding constant.

For the FcRn experiments, the Promega Lumit^{™} FcRn assay was used, which is capable of measuring the interaction between human FcRn and the Fc region of ACE2-Fc constructs. The un-labelled ACE2-Fc competes with human IgG1 labelled with LgBiT (Tracer-LgBiT) for binding to the C-terminally biotinylated human FcRn bound to Streptavidin-SmBiT (hFcRn-Biotin-SA-SmBiT), resulting in a concentration-dependent decrease in luminescent signal with increasing concentration of the ACE2-Fc construct. The generated data were evaluated with a 4PL model fit.

### 5. Analysis of fusion protein glycosylation and sialylation

The ACE2 part and the Fc part of the fusion proteins were separated by digestion with the enzyme IdeS (FabRICATOR^{®}; Genovis) which specifically digests IgG at a site below the hinge region and separation of the two parts on a spin column loaded with CaptureSelect FcXL affinity resin (Thermo Scientific). Specifically, 40 µg of protein sample were mixed with distilled water to a final volume of 80 µL, before 20 µL of digestion buffer (250 mM HEPES, pH 7.2) and 10 µL of FabRICATOR^{®} (40 units) were added. The mixture was incubated for 45 minutes at 37°C.

Afterwards, the digested sample was loaded on a spin column loaded with CaptureSelect FcXL affinity resin and equilibrated with binding buffer (20 mM Tris pH 7.2) and incubated for 30 minutes at room temperature at 750 rpm. Afterwards, the ACE2 fraction was collected by centrifugation (two minutes at 200 g); the flow-through was collected and combined with the fraction of a second centrifugation step after addition of binding buffer. Next, the column was washed with binding buffer (two times) and wash buffer (20 mM sodium acetate, pH 5.0; one time). The Fc part was eluted by adding elution buffer (20 mM acetic acid, pH 3.3), incubating the columns for 5 minutes at 750 rpm and collecting the flow-through by centrifugation (two minutes at 200 g). The elution of the Fc part was repeated twice and the fractions were pooled. The Fc fractions were neutralized to a pH of about 7.0 by adding 3 µL of neutralization buffer (0.5 M sodium phosphate dibasic) per 30 µL sample.

The protein concentration in the ACE2 and Fc samples were determined by measuring the OD280.

The proteins were deglycosylated with RapiGest SF (Waters) dissolved in Glycoworks Rapid Buffer (Waters) to a concentration of 5% (w/v) of which 6 µL were mixed with the ACE2 fraction (2.7 µg of protein diluted to a final volume of 22.8 µL) and the Fc fraction (5.4 µg of protein diluted to a final volumne of 22.8 µL) separately. The samples were heated to 90°C for three minutes and then allowed to cool to room temperature for five minutes. 1.2 µL of Rapid PNGase F (Waters) was added to each tube and the samples were incubated at 50°C for 5 minutes, before they were allowed to cool to room temperature for five minutes.

Glycosylamines were labelled with 12 µL RapiFluor-MS Reagent (23 mg dissolved in 335 µL DMF; Waters) which was added to the deglycosylated samples and mixed. The labelling was allowed to proceed at room temperature for five minutes, before 358 µL acetonitrile were added to each sample.

The labeled glyosylamines were cleaned up using a GlycoWorks HILIC µElution Plate installed on a Waters manifold vacuum system. The wells were conditioned by washing with 200 µL water, three times and equilibrated with 200 µL 85% acetonitrile in water three times. The samples diluted with acetonitrile were loaded in their entirety. The wells were washed twice with 600 µL of 1 :9:90 (v/v/v) formic acid/water/acetonitrile and the waste tray was replaced with a 1 mL 96-well plate. The glycans were eluted with three sequential 30 µL volumes of SPE Elution Buffer (200 mM ammonium acetate in 5% acetonitrile). 310 µL of DMF/acetonitrile solution was added to the 90 µL eluate and mixed by aspiration.

The HILIC analysis was performed as follows:
a) Instrumental parameters:
   - Instrument: Acquity UPLC I-class equipped with FLR detector and Xevo G2-XS QToF mass spectrometer
   - Injection volume: 50 µL
   - Column: ACQUITY UPLC glycan BEH amide, 130Å, 1.7 µm, 2.1×150 mm (Waters)
   - Column temperature: 30°C
   - Gradient of compositions A (50 mM ammonium formate, pH 4.4) and B (acetonitrile) and flow rate: 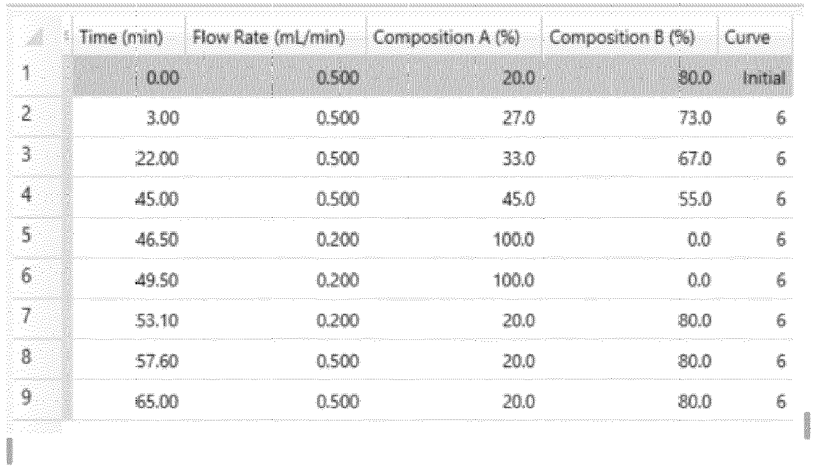
Fluorescence detection settings:
   - Excitation wavelength: 265 nm
   - Emission wavelength: 425 nm
   - Sampling rate: 20 points/sec
   - Filter time constant: 0.10 sec
   - Auto zero: On inject start
   - Auto zero mode: Maintain baseline
   - Gain: 1
   - Data units: Emission
   - Sensitivity: 10000 EUFS
   - Polarity: Positive
   - Voltage offset: 0 mV
   - Chart marks: Enable
   - Rect wave period: 0.2 sec
   - Pulse width: 1.0 sec
Mass spectrometer settings:
   - Scan range 500-2000 m/z
   - Lockspray: 0.25 seconds once every 60 seconds
   - Capillary: 2.75 kV
   - Sample cone: 40 V
   - Source temperature: 120°C
   - Desolvation temperature: 500°C
   - Desolvation gas flow: 800L/h
   - Collision energy: 6 eV

### 6. Neutralization of the SARS-CoV2 Omicron variant

A commercial pseudovirus neutralization assay was used. HEK293 cells stably expressing the full-length ACE2 receptor (Genbank #NM_021804.3) and pseudotyped lentivirus produced with SARS-CoV-2 B.1.1.529 BA.1 variant Spike (Genbank #QHD43416.1 with B.1.1.529 BA.1 mutations) were used. These pseudovirions also contain the firefly luciferase gene driven by a CMV promoter, therefore, the spike-mediated cell entry can be conveniently measured via luciferase reporter activity. ACE2-HEK293 cells (BPS bioscience) were thawed, amplified in growth medium (BPS bioscience), then harvested and plated in white, clear flat bottom 96-well culture plates at 5 ×10³ cells/well in 50 µL of thaw medium. The cells were incubated overnight at 37 °C. The following day, visual control of cell layer homogeneity and integrity was validated using inverted microscope. Test samples containing the ACE2-Fc fusion protein were diluted to reach 12x the expected final concentration in assay wells. The test samples were pre-incubated with Spike (SARS-CoV-2) pseudotyped lentivirus (BPS bioscience) or control bald pseudovirions (BPS bioscience) during approximately 30 minutes at room temperature. Finally, 10 µL of sample-pseudovirus mix were added in the assay wells containing the ACE2-HEK cells in 50 µL of medium. After 24 hours, a complete medium change is operated; the following day a volume of 50 µL of luciferase reagent is added to the assay wells and the luminescent signals are measured using a lumino-fluorimeter. The IC50 was calculated in Prism v5 using a non-linear four parameter regression fit.

Some embodiments of the present invention relate to:
1. A fusion protein comprising:
   (a) a first part comprising a variant of a fragment of human ACE2, wherein the fragment of human ACE2 consists of the amino acid sequence according to SEQ ID NO: 1 and comprises one or more mutations at an amino acid position selected from the group consisting of T92, Q325 and T27, the numbering referring to SEQ ID NO: 67 and
   (b) a second part comprising an Fc portion of a human antibody or a fragment or variant of the Fc portion.
2. The fusion protein according to item 1, wherein the one or more mutations are selected from the group consisting of T92I, Q325P and T27A, the numbering referring to SEQ ID NO: 67.
3. The fusion protein according to item 1 or 2, wherein the first part of the fusion protein consists of the amino acid sequence according to any one of SEQ ID NOs: 13, 14, 15, 16, 17, 18 or 19.
4. The fusion protein according to item 1 or 2, wherein the variant of the human ACE2 fragment further comprises a mutation at amino acid position S645, the numbering referring to SEQ ID NO: 67.
5. The fusion protein according to item 4, wherein the mutation at amino acid position S645 is S645C, the numbering referring to SEQ ID NO: 67.
6. The fusion protein according to any one of items 4 and 5, wherein the first part of the fusion protein consists of the amino acid sequence according to any one of SEQ ID NOs: 20, 21, 22, 23, 24, 25 and 26.
7. The fusion protein according to any one of the preceding items, wherein the first part of the fusion protein is N-glycosylated and 70% to 95% of the N-glycans on the first part have at least one sialic acid molecule attached thereto.
8. Fusion protein according to any one of the preceding items, wherein the Fc portion of a human antibody is the Fc portion of a human IgG1, IgG3 or IgG4 antibody.
9. Fusion protein according to any one of the preceding items, wherein the amino acid sequence of the Fc portion of a human antibody is selected from the group consisting of SEQ ID NO: 2 and SEQ ID NO: 3.
10. Fusion protein according to any one of the preceding items, wherein the Fc portion of a human antibody has the amino acid sequence according to SEQ ID NO: 2 and the first part and the second part of the fusion protein are linked by the sequence according to SEQ ID NO: 12.
11. Fusion protein according to any one of items 1 to 9, wherein the Fc portion of a human antibody has the amino acid sequence according to SEQ ID NO: 3 and the first part and the second part of the fusion protein are linked by the sequence according to SEQ ID NO: 11.
12. Fusion protein according to any one of items 1 to 8, wherein the amino acid sequence of the variant of the Fc portion of a human antibody is selected from the group consisting of SEQ ID NO: 4, SEQ ID NO: 5, SEQ ID NO: 6 and SEQ ID NO: 7.
13. Fusion protein according to any one of items 1 to 8 and 12, wherein the variant of the Fc portion of a human antibody has the amino acid sequence according to SEQ ID NO: 6 or SEQ ID NO: 7 and the first part and the second part of the fusion protein are linked by the sequence according to SEQ ID NO: 12.
14. Fusion protein according to any one of items 1 to 8 and 12, wherein the variant of the Fc portion of a human antibody has the amino acid sequence according to SEQ ID NO: 4 or SEQ ID NO: 5 and the first part and the second part of the fusion protein are linked by the sequence according to SEQ ID NO: 11.
15. The fusion protein according to any one of the preceding claims, wherein the Fc portion of a human antibody or fragment or variant thereof is afucosylated.
16. The fusion protein according to any one of the preceding claims, wherein the second part of the fusion protein is N-glycosylated and 15% to 35% of the N-glycans on the second part have at least one sialic acid molecule attached thereto.
17. Fusion protein according to any one of items 1 to 3 and 7 to 16, wherein the fusion protein has an amino acid sequence selected from the group consisting of SEQ ID NOs: 27, 28, 29, 30, 31, 32, 33, 34, 35, 36, 37, 38, 68, 69, 70, 71, 72 and 73.
18. Fusion protein according to any one of items 5 to 16, wherein the fusion protein has an amino acid sequence selected from the group consisting of SEQ ID NOs: 39, 40, 41, 42, 43, 44, 45, 46, 47, 48, 49, 50, 51, 52, 53, 54, 55, 56, 57, 58, 59, 60, 61, 62, 63, 64, 65, 66, 74, 75, 76, 77, 78, 79, 80, 81, 82, 83, 84, 85, 86 and 87.
19. Nucleic acid molecule comprising a nucleic acid sequence encoding the fusion protein according to any one of items 1 to 18.
20. Expression vector comprising the nucleic acid molecule according to item 19.
21. Host cell comprising the nucleic acid molecule according to item 19 or the expression vector according to item 20.
22. Method for producing the fusion protein according to any one of items 1 to 18, comprising culturing the host cell according to item 21 in a suitable culture medium.
23. Fusion protein according to any one of items 1 to 18 for medical use.
24. Fusion protein according to any one of items 1 to 18 for use in preventing and/or treating an infection with a coronavirus binding to ACE2.
25. Fusion protein for use according to item 24, wherein the coronavirus binding to ACE2 is selected from the group consisting of SARS, SARS-CoV-2 and NL63, preferably it is SARS-CoV-2.
26. Fusion protein for use according to item 24 or 25, wherein the fusion protein is to be administered in combination with an anti-viral agent.
27. Fusion protein for use according to item 22, wherein the anti-viral agent is selected from the group consisting of remdesivir, arbidol HCl, nirmatrelvir, molnupiravir, ritonavir, lopinavir, darunavir, ribavirin, chloroquin and derivatives thereof, nitazoxanide, camostat mesilate, tocilizumab, siltuximab, sarilumab and baricitinib phosphate and combinations of any of the aforementioned.
28. Fusion protein for use according to item 24 or 25, wherein the fusion protein is to be administered in combination with an antibody.
29. Fusion protein for use according to item 28, wherein the antibody is selected from the group consisting of bamlanivimab, etesevimab, casirivimab, imdevimab, sotrovimab bebtelovimab, tixagevimab and cilgavimab and mixtures thereof.
30. Fusion protein according to any one of items 1 to 18 for use in treating hypertension (including high blood pressure), congestive heart failure, chronic heart failure, acute heart failure, contractile heart failure, myocardial infarction, arteriosclerosis, kidney failure, renal failure, Acute Respiratory Distress Syndrome (ARDS), Acute Lung Injury (ALI), chronic obstructive pulmonary disease (COPD), pulmonary hypertension, renal fibrosis, chronic renal failure, acute renal failure, acute kidney injury, inflammatory bowel disease and multi-organ dysfunction syndrome.
31. Pharmaceutical composition comprising the fusion protein according to any one of items 1 to 18 and a pharmaceutically acceptable carrier or excipient.
32. Pharmaceutical composition according to item 31, further comprising an anti-viral agent and/or an antibody.
33. Method for producing the fusion protein according to any one of items 7 to 18, comprising culturing a eukaryotic host cell comprising a recombinant nucleic acid molecule encoding said fusion protein in a medium supplemented with one or more of a manganese salt, N-acetylmannosamine or a derivative thereof, galactose, glucosamine or a derivative thereof and DMSO.
34. Method according to item 33, wherein the eukaryotic host cell is cultured in a medium supplemented with 20 to 80 µM manganese chloride.
35. Method according to item 33 or 34, wherein the eukaryotic host cell is cultured in a medium supplemented with 5 to 30 mM N-acetylmannosamine.
36. Method according to any one of items 33 to 35, wherein the eukaryotic host cell is cultured in fed-batch mode.
37. Method according to item 36, wherein the eukaryotic host cell is fed with manganese chloride and galactose.
38. Method for producing the fusion protein according to any one of items 15 to 18, comprising culturing a eukaryotic host cell comprising a recombinant nucleic acid molecule encoding said fusion protein in a medium supplemented with a fucosylation inhibitor.
39. Method according to item 38, wherein the fucosylation inhibitor is 2-fluorofucose.
40. Method for producing the fusion protein according to any one of items 15 to 18, comprising culturing a eukaryotic host cell comprising a recombinant nucleic acid molecule encoding said fusion protein, wherein the eukaryotic host cell is genetically modified to decrease the activity of a protein involved in fucosylation.
41. Method according to item 40, wherein the protein involved in fucosylation is selected from the group consisting of GDP-mannose 4,6-dehydratase (GMD), GDP-fucose transporter (GFT) and alpha-(1,6)-fucosyltransferase (FUT8).
42. Method according to any one of items 33 to 41, wherein the eukaryotic host cell is a CHO cell.

## Claims

1. A fusion protein comprising:
(a) a first part comprising a variant of a fragment of human ACE2, wherein the fragment of human ACE2 consists of the amino acid sequence according to SEQ ID NO: 1 and wherein the variant of the human ACE2 fragment comprises one or more mutations at an amino acid position selected from the group consisting of T92, Q325 and T27, the numbering referring to SEQ ID NO: 67 and
(b) a second part comprising an Fc portion of a human antibody or a fragment or variant of the Fc portion.

2. The fusion protein according to claim 1, wherein the one or more mutations are selected from the group consisting of T92I, Q325P and T27A, the numbering referring to SEQ ID NO: 67.

3. The fusion protein according to claim 1 or 2, wherein the variant of the human ACE2 fragment further comprises a mutation at amino acid position S645, the numbering referring to SEQ ID NO: 67.

4. The fusion protein according to claim 3, wherein the mutation at amino acid position S645 is S645C, the numbering referring to SEQ ID NO: 67.

5. The fusion protein according to any one of the preceding claims, wherein the first part of the fusion protein consists of the amino acid sequence according to any one of SEQ ID NOs: 13, 14, 15, 16, 17, 18, 19, 20, 21, 22, 23, 24, 25 or 26.

6. The fusion protein according to any one of the preceding claims, wherein the first part of the fusion protein is N-glycosylated and 70% to 95% of the N-glycans on the first part have at least one sialic acid molecule attached thereto.

7. The fusion protein according to any one of the preceding claims, wherein the second part of the fusion protein has an amino acid sequence according to any one of SEQ ID NOs: 4, 5, 6 or 7.

8. The fusion protein according to any one of the preceding claims, wherein the Fc portion of a human antibody or fragment or variant thereof is afucosylated.

9. The fusion protein according to any one of the preceding claims, wherein the Fc part of the fusion protein is N-glycosylated and 15% to 35% of the N-glycans on the Fc part have at least one sialic acid molecule attached thereto.

10. The fusion protein according to any one of the preceding claims, wherein the fusion protein has an amino acid sequence according to any one of SEQ ID NOs: 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65 or 66.

11. The fusion protein according to any one of claims 1 to 10 for medical use.

12. The fusion protein according to any one of claims 1 to 10 for use in preventing and/or treating an infection with a coronavirus binding to ACE2, preferably wherein the coronavirus binding to ACE2 is selected from the group consisting of SARS, SARS-CoV-2 and NL63, preferably it is SARS-CoV-2.

13. Pharmaceutical composition comprising the fusion protein according to any one of claims 1 to 10 and a pharmaceutically acceptable carrier or excipient.

14. Method for producing the fusion protein according to any one of claims 6 to 10, comprising culturing a eukaryotic host cell comprising a recombinant nucleic acid molecule encoding said fusion protein in a medium supplemented with one or more of a manganese salt, N-acetylmannosamine or a derivative thereof, galactose, glucosamine or a derivative thereof and DMSO.

15. Method for producing the fusion protein according to any one of claims 8 to 10, comprising culturing a eukaryotic host cell comprising a recombinant nucleic acid molecule encoding said fusion protein in a medium supplemented with a fucosylation inhibitor, preferably wherein the fucosylation inhibitor is 2-fluorofucose.
